(19) Europäisches Patentamt
European Patent Office
Office européen des brevets

(11) EP 1 210 042 B1

(12) **EUROPÄISCHE PATENTSCHRIFT**

(45) Veröffentlichungstag und Bekanntmachung des Hinweises auf die Patenterteilung:
**18.06.2008 Patentblatt 2008/25**

(21) Anmeldenummer: **00956020.2**

(22) Anmeldetag: **11.09.2000**

(51) Int Cl.:
***A61F 9/01*** *(2006.01)* ***A61B 3/10*** *(2006.01)*

(86) Internationale Anmeldenummer:
**PCT/CH2000/000488**

(87) Internationale Veröffentlichungsnummer:
**WO 2001/019303 (22.03.2001 Gazette 2001/12)**

# (54) VORRICHTUNG ZUR FOTOABLATION DER KORNEA MIT EINEM LASERSTRAHL

DEVICE FOR THE PHOTOABLATION OF THE CORNEA WITH A LASER BEAM

DISPOSITIF DE PHOTOABLATION DE LA CORNEE PAR RAYONNEMENT LASER

(84) Benannte Vertragsstaaten:
**AT BE CH CY DE DK ES FI FR GB GR IE IT LI LU MC NL PT SE**

(30) Priorität: **10.09.1999 CH 166199**

(43) Veröffentlichungstag der Anmeldung:
**05.06.2002 Patentblatt 2002/23**

(73) Patentinhaber: **HAAG-STREIT AG**
**CH-3098 Köniz (CH)**

(72) Erfinder: **WAELTI, Rudolf**
**CH-3097 Liebefeld (CH)**

(74) Vertreter: **Roshardt, Werner Alfred**
**Keller & Partner**
**Patentanwälte AG**
**Schmiedenplatz 5**
**Postfach**
**3000 Bern 7 (CH)**

(56) Entgegenhaltungen:
**DE-A- 19 704 602**
**DE-C- 19 635 998**
**US-A- 5 490 849**
**US-A- 5 493 109**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

### Technisches Gebiet

Die Erfindung betrifft eine Vorrichtung zur Fotoablation der Komea mit einem Laserstahl, mit dem eine Vielzahl hintereinander folgender Teilablationsvorgänge vorgenommen werden sowie eine Vorrichtung mit einer Laserstrahlungsquelle zur Durchführung dieses Verfahrens. Mit der Erfindung können intraokulare Dicken und Distanzen im vorderen Augenabschnitt unmittelbar vor, während und unmittelbar nach chirurgischen Eingriffen und Behandlungen der Kornea gemessen werden. Abhängig von diesen Messergebnissen kann eine Steuerung einer photorefraktiven Behandlung in Echtzeit erfolgen. Die Steuerung in Echtzeit führt zu einer erhöhten Sicherheit für den Patienten und einer verbesserten Genauigkeit der photorefraktiven Augenkorrektur.

### Stand der Technik

In der US-A 5 720 894 ist eine Materialabtragung mittels Laserstrahlen von biologischem Gewebe, insbesondere von hartem Gewebe sowie einer Komea beschrieben. Hierzu konnten gepulste Abtragungs-Laser mit einer Wellenlänge von 200 nm bis 2'000 nm, einer Pulsbreite von 0,02 ps bis 100 ps und einer Repetitionsrate von 10 Hz bis 2'000 Hz verwendet werden. Eine exakte Abtragung wurde erreicht, indem immer nur ein kleines Volumen abgetragen wurde. Das mit den Laserpulsen abgetragene Material konnte spektroskopisch oder mittels OCT (optical coherence tomography) ermittelt werden. Beim OCT wurde die Tiefe des Abtragungskraters ausgemessen. In der US-A 5 720894 wurde zwar eine Abtragungsrate mittels OCT, jedoch kein Dickenprofil ermittelt.

In der US-A 5 693109 wurde bei einer Katarakt-Chirurgie und einer refraktiven Chirurgie die erreichte Brechkraft der Linse, mittels OCT ausgemessen. Mit der Messung wurde die vordere und die hintere Oberflächenkontur sowie die Dicke der Komea unter Verwendung einer Laserwellenlänge von 850 nm bestimmt. Eine Verwendung eines Lasers zur gesteuerten Bearbeitung der Komea ist jedoch der US-A 5 693 109 fremd.

In der DE-A 197 04 602 ist ein Messsystem, welches nach dem Prinzip eines Michelson-Interferometers arbeitete, beschrieben. Alle optischen Wege des Interferometers verliefen in Luft. Eine Verwendung von Strahlungsleitem, weiche die ganze Messanordnung erst handlich machen würden, ist der DE-A 197 04 602 fremd.

### Darstellung der Erfindung

### Aufgabe der Erfindung

Aufgabe der Erfindung ist es, eine Vorrichtung zu schaffen, mit denen eine sichere Bearbeitung der Kornea möglich ist.

### Lösung der Aufgabe

Die Aufgabe wird dadurch gelöst, dass vor und/ oder nach jedem Teilablationsvorgang mit einer mit einem Michelson-Interferometer zusammenarbeitenden Messeinrichtung die Dicke der Kornea und nicht nur deren Oberflächenform und deren optische Brechkraft bestimmt wird. Zudem wird die Lage des für die Ablation vorgesehenen Bereichs ermittelt und eine Freigabe zur Ablation nur gegeben, sofern der Bereich sich innerhalb vorgegebener Toleranzwerte befindet. Fehlerhafte Ablationsvorgänge, wie sie sich beispielsweise durch eine Augenbewegung ergeben können, sind hierdurch ausgeschlossen.

Die Messeinrichtung führt dann infolge der ermittelten Dickenwerte der Kornea den Laserstrahl mit nachgeregelter Intensität gesteuert über dickenmäßig noch zu korrigierende Bereiche der Kornea. Die Teilablationen werden dadurch derart vorgenommen, dass unter Beachtung eines vorgegebenen Korneaprofils eine vorgegebene Korneadicke nicht unterschritten wird.

Vorrichtungsmäßig wird hierzu eine Laserstrahlungsquelle, eine Steuer- und eine Messeinrichtung vorgesehen. Die Steuereinrichtung *kann* eine.Strahlablenkeinheit *haben,* mit der der Laserstrahl transversal über die Kornea führbar ist. Die erfindungsgemäße Vorrichtung hat ferner eine Messeinrichtung mit einem Michelson-Interferometer mit einer Zentrumswellenlänge der Messstrahlungsquelle im Bereich von 1'310 nm und einer Monomode-Faser für diese Wellenlänge im Referenzarm, damit die optische Weglänge in Luft im Messarm, welche für die Durchführung der Messung notwendig ist, einwandfrei kompensierbar ist.

Wie unten beschrieben ist, sind in der Steuereinrichtung diverse Eichkurven betreffend einer Bestimmung des optimalen Abstandes des Behandlungsortes sowie dessen Neigungstoleranzen zur behandelnden Laserstrahlachse abgelegt.

Durch die Verwendung einer Messstrahlungsquelle im Bereich von 1'310 nm mit einer Bandbreite mit bis ca. +/- 100 nm werden insbesondere signalverzerrende und -vermindernde Folgen der Dispersion minimiert. Wird nämlich eine Strahlungsquelle mit einer beliebig vorgegebenen Zentrumswellenlänge benutzt, so besteht im Messarm infolge des durch das Operationsmikroskop, die Funduskamera oder den Laser vorgegebenen Aufbaus im Allgemeinen ein relativ langer Weg in Luft in der Größenordnung eines Meters. Dieser Weg in Luft muss im Referenzarm entweder durch einen entsprechenden Weg in Luft oder in einem anderen Medium (vorzugsweise in einer Monomodefaser aus Platzersparnisgründen) kompensiert werden, damit die erforderlichen Interferenzsignale erzeugt werden können. Wird der im Messarm in Luft zurückgelegte Weg im Referenzarm mit einer entsprechenden Strecke in Luft kompensiert, so entsteht durch die Dispersion zwar nur eine geringe Signalverzerrung und Signalverminderung. In diesem Fall kann aber der Referenzarm nicht mehr kompakt gebaut werden. Wird der im Messarm in Luft zurückgelegte Weg jedoch mit einer Monomodefaser im Referenzarm kompensiert, entsteht normalerweise eine relativ hohe Signalverzerrung und Signalverminderung, weil die Dispersionseigenschaften im Referenz- und im Messarm unterschiedlich sind. Es gibt jedoch erstaunlicherweise einen Wellenlängenbereich um 1'310 nm herum, wo diese Signalverzerrung und Signalverminderung minimiert ist, so dass der optische Weg eines Messarmes in Luft von mehreren Metern durch einen in einer Monomodefaser zurückgelegten Weg im Referenzarm kompensiert werden kann, ohne dass das Interferenzsignal dadurch gestört wird. Durch die Kompensation eines optischen Weges in einer Faser kann das Interferometer äusserst kompakt und billig gebaut werden.

In einer bevorzugten Ausführungsform können auch die Laserparameter wie Durchmesser auf der Kornea, Intensität, Leistung, Energie und Pulsdauer der Laserstrahlung so gesteuert werden, dass Einflüsse der Umwelt auf das Ergebnis der Photorefraktion wie Luftfeuchtigkeit und Lufttemperatur im Operationsraum, sowie patientenspezifische Einflüsse wie Verdunstung von im Operationsraum gelagerten Chemikalien [z. B. (offene) Alkoholbehälter, (offene) Putzmittelbehälter], Alter, Geschlecht, Zusammensetzung der Kornea des Patienten und Temperatur dessen Kornea berücksichtigt werden können. Es könnten auch patientenspezifische Daten berücksichtigt werden.

Um dies zu ermöglichen, werden in vorgängigen Untersuchungen die Korneadicke als Funktion der Luftfeuchtigkeit, Lufttemperatur im Operationsraum, Alter, Geschlecht, Zusammensetzung der Kornea und Temperatur der Kornea gemessen. Aus den Ergebnissen dieser Messungen werden dann Korrekturfaktoren ermittelt, welche in die Steuerung des Laserstrahls eingehen. Es kann dann auch bei Abweichungen von den Normalbedingungen ein ausgezeichnetes Ergebnis erzielt werden. Es können sogar bei Patienten mit irregulärem Astigmatismus, Keratokonus oder irregulärer, gewellter Korneaoberfläche photorefraktive Korrekturen vorgenommen werden, welche die durch die optischen Beugungsgesetze gegebene Grenze von ca. 20/5 - 20/8 (je nach Pupillengröße) erreichen.

Mit der hier beschriebenen Messanordnung ist es erstmals möglich, die Korneavorderfläche, die Kornearückfläche, die Distanz der Kornea zu einem Referenzort, die Neigung der Kornea und somit die Koordinaten der gesamten Kornea im dreidimensionalen Raum online während des gesamten refraktiven chrirugischen Eingriffs und zudem noch kontaktlos zu bestimmen.

## Kurze Beschreibung der Zeichnungen

Nachfolgend werden Beispiele der erfindungsgemäßen Vorrichtung sowie des erfindungsgemäßen Verfahrens anhand der Zeichnungen näher erläutert. Es zeigen:

Fig. 1 ein Blockschema der erfindungsgemäßen Vorrichtung;

Fig. 2 eine Messeinrichtung der in **Figur 1** dargestellten Vorrichtung mit einem 2x2 Faserkoppler;

Fig. 3 eine Variante zu der in **Figur 2** dargestellten Messeinrichtung mit einem 3x3 Faserkoppler;

Fig. 4 eine Variante zu der in **Figur 2** dargestellten Messeinrichtung mit zwei 2x2 Faserkopplern;

Fig. 5 einen Strahlengang des Messstrahls, bei dem dieser immer annähernd senkrecht auf das Messobjekt trifft;

Fig. 6 eine beispielsweise Anordnung des Messarmes mit einer polarisationserhaltenden Faser;

Fig. 7 ein Flussdiagramm, welches ein Verfahren bei der Abtragung für LASIK darstellt;

Fig. 8 ein Flussdiagramm, welches ein Verfahren bei der Abtragung für PRK (**p**hoto**r**efraktive **K**eratektomie) darstellt;

Fig. 9 eine Ausführungsvariante, welche eine periphere Korneadickenmessung oder Skleradikkenmessung ermöglicht;

Fig. 10 eine Variante zu der in **Figur 9** dargestellten Ausführungsform;

Fig. 11 eine weitere Variante mit einer Messstrahlungseinkopplung in ein Stereomikroskop;

Fig. 12 eine schematische Anordnung zur Bestimmung des wassergehalts in der Kornea und

Fig. 13 eine Variante zu der in **Figur 12** dargestellten Anordnung.

## Wege zur Ausführung der Erfindung

Die nachfolgend beschriebene Erfindung wird vorzugsweise in der Augenbehandlung eingesetzt. Das menschliche Auge ist ein äußerst empfindliches Objekt, welches sich zudem in seiner Lage schlecht fixieren lässt. Es werden deshalb zusammen mit dem Materialablationsvorgang Methoden zur Lagebestimmung des Auges angegeben, wobei die Ablation, welche mit einem Laser vorgenommen wird, immer nur dann vonstatten gehen soll, wenn die Lage des zu behandelnden Augenbereiches optimal ist. Es kann sich nämlich einmal der Abstand des zu behandelnden Bereichs zu einer Fokussieroptik für die Laserstrahlung geändert haben. Auch kann der Patient das Auge geringfügig bewegt haben, so dass die Achse der bearbeitenden Strahlung nicht mehr senkrecht auftreffen würde. D. h. Materialablation und Lagebestimmung sind miteinander verknüpft. Eine Ablation kann nicht durchgeführt werden, falls die Bereichslage nicht ausreichend gut ist. Der Ablationsvorgang wird bei einer Lageabweichung automatisch blokkiert.

Bei einer Abstandsmessung wird, wie bereits oben angedeutet, die Distanz zwischen der Vorderfläche der Komea und einem definierten Referenzort ermittelt. Der Referenzort kann ein vom Behandlungslaser aus vorgegebener Ort oder ein vom Messgerät vorgegebener Ort sein. Ein vom Behandlungslaser bestimmter Ort ist z. B. die Koordinate des optimalen Behandlungsortes auf der Kornea. Ein vom Messgerät ausgezeichneter Ort ist eine definierte Position des unten verwendeten Weglängenvariators im Michelson-Interferometer. Diese Position kann durch einen sogenannten Encoder angegeben werden.

Eine Abstandseichung wird man für jedes Behandlungsgerät vor seiner Inbetriebnahme vornehmen. Wird z. B. ein rotierender Würfel in einem Michelson-Interferometer zur Abstandsbestimmung eingesetzt, wie er beispielsweise in der WO 99/22198 beschrieben ist, so ist ein besonders ausgezeichneter Ort die Nullgradposition des Würfels. In diesem Fall liegt die hochreflektierend beschichtete Fläche des Würfels genau senkrecht zum einfallenden Referenzarmstrahl. Das bei dieser Nullgradposition in die Referenzarmfaser rückreflektierte Licht wird von der Detektionselektronik gemessen. Der Encoder wird jetzt so eingestellt, dass er genau bei dieser Nullgradposition einen elektrischen Puls abgibt. Die zeitliche Zuordnung dieses Referenzpulses mit einem Reflexionspuls von der Vorderfläche der Komea kann zu Distanzmessungen verwertet werden. Eine Eichung erfolgt über ein im optimalen Behandlungsort reflektierendes Objekt. Dieser Abstandswert wird vor Inbetriebnahme des Behandlungsgeräts abgespeichert und dient als Vergleichswert bei nachfolgenden Behandlungen.

Als Meßobjekt kann man eine im optimalen Behandlungsort justierte Glasplatte verwenden. Ein optimaler Ort kann beispielsweise unter Verwendung zweier Pilotlaserstrahlen ermittelt werden, wobei dann die Glasplatte sich am Ort der sich kreuzenden Strahlen angeordnet sein sollte.

Abgesehen vom richtigen Abstand muss auch eine richtige Lage des zu behandelnden Bereichs erreicht werden. Die richtige Lage wurde bisher mit Videokameras überprüft. Das aufgenommene Videobild wurde auf einem Bildschirm wiedergegeben und dort mit einer vorgegebenen angezeichneten Lageposition verglichen. Hiermit konnte zwar eine Lagekontrolle vorgenommen werden, ein Verkippen des Auges durch eine geänderte Blickrichtung konnte jedoch nicht erfasst werden. Es konnten deshalb bei z. B. Fixationsschwierigkeiten des Patienten ungewollt dezentrierte Ablationsmuster "geschossen" werden. Solche Dezentrierungen können zu ungenauen Korrekturen der Sehkraft führen, welche eventuell sogar eine Nachbehandlung nötig machen. Durch die nachfolgend beschriebenen Vorkehrungen ist es möglich, anhand der mit der Messvorrichtung gemessenen Signalhöhen der Vorder- und Rückflächen der Komea deren Neigung online während der refraktiven Chirurgie zu messen und anhand der Messdaten gegebenenfalls das Ablationsmuster anzupassen.

Die Höhe der elektrischen Detektionssignale von der Vorder- und der Rückfläche sind von der Empfindlichkeit der Messvorrichtung, von der numerischen Apertur des Messstrahls, von der auf die Komea auftreffenden optischen Messlichtleistung, vom Brechungsindizes der Kornea und der Vorderkammer sowie von der Neigung der Komea zum Messstrahl abhängig. Die Empfindlichkeit der Messvorrichtung, die numerische Apertur des Messstrahls und die auf die Komea auftreffende Messlichtleistung sind bekannt und konstant. Der Brechungsindex der Komea und der Vorderkammer schwankt bei verschiednen Patienten nur in einem kleinen Bereich. Es hängt deshalb die Höhe der gemessenen Signale fast nur von der Neigung der Komea bezüglich des Messstrahls ab. Man wird deshalb an verschiedenen Versuchspersonen vorgängig Kalibrierungsmessungen vornehmen und diese in Abhängigkeit des Neigungswinkels abspeichern. Bei einer Ablationsbehandlung am Patienten können diese Eichwerte dann mit den Ist-Werten verglichen werden, woraus sich eine aktuelle Neigung ermitteln lässt. Ist der derart ermittelte Neigungswinkel zu groß, wird die Behandlung unterbrochen.

Das in Figur 1 dargestellte Blockschema der erfindungsgemäßen Vorrichtung hat eine unten in mehreren Varianten beschriebene Messeinrichtung 1, eine Steuereinrichtung 3 sowie eine Laserstrahlungsquelle 7. Die Steuerelektronik 3 hat einen mit einer Auswerteeinheit zusammenwirkenden Datenspeicher und eine Treibereinheit. Die Laserstrahlung der Strahlungsquelle 7 wird über einen auch als Strahlteiler wirkenden Umlenkspiegel 5 und eine Optik 6 auf einen auf der Kornea zu behandelnden Bereich berichtet. Der zu behandelnde Bereich ist mit dem Strahl eines Fixierlasers 9 markierbar. Das Auge und die genaue seitliche Lage des zu behandelnden Bereichs wird wie bereits oben ausgeführt, mit einer Infrarot-Kamera 11 als Auswerteeinheit überwacht.

Die Laserstrahlungsquelle 7 wird je nach den gewünschten chirurgischen Techniken [PRK (photorefraktive Keratomie), PTK (phototherapeutische Keratomie), LTK (Laserthermokeratoplastie), LASIK (Laser-assisted in situ Keratomileusis), LASEK (Laser Epithelial Keratomileusis)] am Auge **25** ausgewählt. So wird man zum Abtragen von Teilen der Komea **24** einen Excimer-Laser verwenden und zu deren Erwärmung einen Holmium-Laser. LASEK basiert auf der Ablösung des Epithels oder eines epithelialen Flaps durch eine alkoholische Lösung. Das abgelöste Ephithel wird mit einer Art Spachtel auf die Seite weggerollt. Das Laser schießt dann direkt auf das offene Stroma der Komea und trägt es bis zur gewünschten Form ab. Danach wird das Epithel oder der epitheliale Flap an seinen früheren Platz zurückgerollt. Eine weiche Kontaktlinse wird aufgesetzt und ein paar Tage getragen, damit das repositionierte Epithel bzw. der epitheliale Flap sich nicht verschiebt.

In **Figur 2** ist eine beispielsweise Messeinrichtung 1 dargestellt. Die Messeinrichtung **1** ist in Anlehnung an ein Michelson-Interferometer aufgebaut. Sie hat eine Strahlungsquelle **26.1** mit vorzugsweise kurzer Kohärenzlänge (typisch 15 μm bis 30 μm), deren Strahlung in einen 2x2 Faserkoppler **29.1** eingekoppelt wird. Die Kohärenzlänge ist kurz gegenüber der zu bestimmenden Dicke der Kornea. Der Faserkoppler **29.1** hat zwei Eingänge und zwei Ausgänge. Er teilt die Strahlung in zwei Teile auf. Der eine Teil der Strahlung wird in einen Referenzarm **30** mit einer vereinfacht als Linse dargestellten Einkopplungsoptik **37.1 a** und einem optischen Weglängenvariator **32.1** eingestrahlt. Der optische Weglängenvariator **32.1** verursacht eine zeitliche Änderung der optischen Weglänge im Referenzarm **30** um eine Strecke ΔZ. Die Einkopplungsoptik **37.1a** formt die aus dem Ende **27** des einen vom Faserkoppler **29.1** ausgehenden Strahlungsleiters **28** austretende Strahlung in einen parallelen Freiraumstrahl **22** um. Der Messarm **31** weist ferner einen Polarisationskontroller **33.1,** eine Monomodefaser 35 mit zwei Fasersteckern **36.1a** und **36.1b,** eine Einkopplungsoptik**37.1b** und eine optische Einheit **39.1** auf, welche speziell für einen vorgegebenen Behandlungszweck mit der Laserstrahlungsquelle **7** *(Behandlungslaser)* ausgebildet ist. Zur optischen Einheit **39.1** kann die in **Figur 1** dargestellte Strahlablenkeinheit **19** gehören. Die Einkopplungsoptik **37.1 b,** welche vereinfacht in **Figur 1** als Strahlteiler **5** dargestellt ist, kann z.B. aus einem Linsensystem und einem Strahlungsteiler bestehen. Der Polarisationskontroller **33.1** ist notwendig, um insbesondere die Polarisationsrichtung der vom Messobjekt **24,** hier der menschlichen Komea **24.1** des Auges **25,** reflektierten Strahlung an die Polarisationsrichtung der im Weglängenvariator **32.1** reflektierten Strahlung derart anzugleichen, dass ein durch eine Strahlungsüberlagerung im Faserkoppler **29.1** erzeugtes Interferenzsignal maximale Intensität erhält. Der Polarisationskontroller **33.1** kann statt im Messarm **31** auch im Referenzarm **30** angeordnet sein. Die vom Referenzarm **30** im Weglängenvariator **32.1** und vom Messobjekt**24.1** reflektierte Strahlung wird im Faserkoppler **29.1** überlagert und ein partieller Strahlungsteil zu einer Photodiode **40** geleitet. Von der Photodiode **40** wird das elektrische Signal zu einer elektronischen Signalverarbeitungseinheit **41** geführt Die Einheit **41** besteht beispielsweise aus einer elektronischen Verstärkerstufe, einer elektronischen Filterung, einem Gleichrichter und einem Tiefpassfilter zur Erzeugung eines Enveloppensignales. Die durch die Einheit **41** ermittelten Daten und Werte werden dann an die in **Figur 1** befindliche Infrarot-Kamera 11 als Auswerteeinheit, von dieser an den Datenspeicher **13** weitergeleitet und für eine weitere Bearbeitung abgespeichert.

In der Einkopplungsoptik **37.1 b** kann, wie bereits oben angedeutet, optional ein (nicht dargestelltes) optisches transversales Strahlablenksystem eingebaut sein. Mit Hilfe dieses Strahlablenksystems ist es möglich, sowohl die Topographie als auch die Tomographie der Komea **24** (z.B. Komeadicke an mehreren transversalen Punkten) zu erfassen und dann auf einem Bildschirm darzustellen. Dieses Strahlablenksystem bewirkt, dass der Messstrahl auf der Komea **24** von der Zentrumsposition ausgelenkt wird. Damit kann bei kleinen Auslenkungen des Strahlablenksystems die Komeadicke an den dem Zentrum benachbarten Punkten gemessen werden. Hierdurch wird auch bei Patienten mit hohem Astigmatismus oder Keratokonus ein möglichst gutes Interferenzsignal in der Zone der höchsten Abtragung erhalten. Bei größeren Auslenkungen des Strahlablenksystems kann die Komeadicke an peripheren Punkten der Komea gemessen werden.

Beispiele für Ablenksysteme **19** sind:

1) zwei bewegliche Spiegel für zwei transversale Ablenkrichtungen

2) ein einziger beweglicher Spiegel, der derart bewegt werden kann, dass damit beide transversalen Ablenkrichtungen ausgeführt werden können. [Vorteile gegenüber 1) sind Platzersparnis und weniger Komponenten.]

3) eine Linse, die transversal (horizontal und vertikal) bewegt werden kann. [Vorteile gegenüber 2): Platzersparnis und weniger Komponenten.]

Um den transversal ausgelenkten freien Messstrahl **44** immer annähernd senkrecht auch auf dem peripheren Bereich der Kornea **24** auftreffen lassen zu können, kann beispielsweise der Messstrahl **44** unmittelbar vor dem Patientenauge mit einem speziell geformten Spiegel **45.1** abgelenkt werden. Dieser speziell geformte Spiegel **45.1** kann beispielsweise, wie in **Figur 2** angedeutet, ein verspiegelter Hohlzylinder **45,1** (mit einem links und rechts offenen Ende) oder ein anderer Hohlkörper mit zwei Öffnungen sein, der vor dem Patientenauge **25** positioniert ist. Ein derartiger Spiegel **45.4** ist unten an Hand der **Figur 5** erläutert.

**Figur 3** zeigt eine zu **Figur 2** analoge Messeinrichtung **1.2.** Es ist lediglich ein 3x3 Faserkoppler **29.2** anstelle des Faserkopplers **29.1** der **Figur 2** und statt der einen Photodiode **40** sind zwei Photodioden **47a** und **47b** vorhanden. Der Faserkoppler **29.2** ist ein 3x3 Faserkoppler mit drei Eingängen und drei Ausgängen. Mit Hilfe des 3x3 Faserkopplers **29.2** wird nun das Interferenzsignal der im dortigen Mess- und Referenzarm **49a** und **49b** reflektierten Strahlung auf zwei Photodioden **47a** und **47b** geführt. Mit einer entsprechenden Auswertung der elektronischen Signale der beiden Photodioden **47a und 47b** in einer zur Signalverarbeitungseinheit **41** analog ausgebildeten Signalverarbeitungseinheit **50.1** ist gegenüber nur einer Photodiode **40** wie in **Figur 2** eine wesentliche Reduzierung des Rauschens möglich (z. B. Intensitätsrauschen der Strahlungsquelle).

In **Figur 4** ist eine Messeinrichtung **1.3** dargestellt, welche zwei 2x2 Faserkoppler **53a** und **53b** hat. Mit dieser Messeinrichtung **1.3** ist gegenüber den Messeinrichtungen **1.1** und **1.2** ein größerer Rauschanteil eliminierbar.

Von einer zu den Strahlungsquellen **26.1** und **26.2** analog ausgebildeten Strahlungsquelle **26.3** geht die Messstrahlung auf den Faserkoppler **53b** und wird hier in den Messarm **54** und einen Referenzarm **55** aufgeteilt. Der Referenzarm **55** hat den Weglängenvariator **32.3,** die beiden Strahlungsleiter **56a** und **56b** sowie die beiden Kollimationseinheiten **57a** und **57b.** Im Gegensatz zu den Messeinrichtungen **1.1** und **1.2** der **Figuren 2** und **3** wird nun der Strahl **59** nicht wieder in denselben Strahlungsleiter **56a** zurückgeführt, sondern nach einer Fokussierung durch eine Kollimatoreinheit **57b** in einen Strahlungsleiter **56b** eingekoppelt, der mit dem Faserkoppler **53a** verbunden ist.

Die Strahlung im Messarm **54,** der analog zu den Ausgestaltungen in den Messanordnungen **1.1** und **1.2** ebenfalls einen Polarisationskontroller **33.3,** eine Einkopplungsoptik **37.3,** eine optische Einheit **39.3** und einen speziell ausgebildeten Spiegel **45.3** hat, wird ebenfalls an einer auszumessenden Kornea **24.3** reflektiert und läuft dann im Messarm **54** zurück bis zum Faserkoppler **53b.** Die Strahlung gelangt dann, je nach verwendetem Verfahren, durch einen Strahlungsleiter **61** zum Faserkoppler **53a.** Im Faserkoppler **53a** erfolgt dann eine interferierende Überlagerung der ankommenden Strahlungen der Strahlungsleiter **56b** und **61.** Die interferierende Strahlung wird einmal über einen Strahlungsleiter **62a** auf eine Strahlungsdetektionseinheit **63a** und ein weiteres Mal über einen Strahlungsleiter **62b** auf eine Strahlungsdetektionseinheit **63b** geführt. Die elektrischen Detektionssignale der beiden Einheiten **63a** und **63b** werden auf eine analog zur Signalverarbeitungseinheit **50.1** ausgebildeten Signalverarbeitungseinheit **50.2** geführt und durch diese verarbeitet. Eine weitere Verarbeitung zu einer der unten genannten Verwendungen erfolgt dann mit einer mit der Einheit **50.2** signalmäßig verbundenen Auswerteeinheit **64,** welche bevorzugtermaßen die notwendigen Datenspeicher enthält.

Der Vorteil der in **Figur 4** gezeigten Schaltung besteht darin, dass durch die Verwendung der beiden Strahlungsdetektionseinheiten **63a** und **63b** der Rauschanteil (z. B. Intensitätsrauschen der Strahlungsquelle **26.3)** verringert werden kann. Die beiden Strahlungsdetektionseinheiten **63a** und **63b** werden antiparallel geschaltet, damit der für die Auswertung unbrauchbare Teil des auf die eine Einheit auftreffenden Strahlungsstromes ein positives elektrisches Stromsignal erzeugt, während der für die Auswertung unbrauchbare Teil des auf die andere Einheit auftreffenden Strahlungsstromes ein negatives elektronisches Stromsignal erzeugt. Die beiden Stromsignale werden zusammengeführt und heben sich dadurch auf. Der für die Auswertung brauchbare Teil (d. h. das Interferenzsignal, hier ein zeitlich begrenzter Sinuszug) der auf die eine Strahlungsdetektionseinheit auftreffenden Strahlung hat eine Phasenverschiebung von $\Pi$ gegenüber dem Interferenzsignal, welches auf die andere Strahlungsdetektionseinheit auftrifft. Werden zwei zeitlich begrenzte Sinuszüge phasenverschoben und voneinander subtrahiert (infolge der antiparallelen Schaltung der beiden Strahlungsdetektionseinheiten **63a** und **63b),** so entsteht wieder ein Sinuszug. Im Gegensatz zum Rauschen entsteht beim Interferenzsignal durch diese antiparallele Schaltung keine Auslöschung. Ergänzend sei bemerkt, dass dieses Prinzip auch bei den Fotodioden **47a** und **47b** in der in **Figur 3** dargestellten Anordnung verwendet wird, wobei hier jedoch wegen der Verwendung eines 3 x 3 Faserkopplers **29.2** die Phasen der Interferenzsignale zueinander nicht um $\pi$, sondern um 2/3 $\pi$ verschoben sind.

**Figur 5** zeigt den Strahlengang des Messstrahls für einen unabgelenkten, freien Strahl **UB** (punktiert) in der optischen Achse **65,** einen durch eine Strahlablenkeinheit **67** aus der optischen Achse **65** leicht ausgelenkten Strahl **SDB** (gestrichelt) und einen durch die Strahlablenkeinheit **67** einer analog zu den Einkopplungseinheiten **37.1** bis **37.3** ausgebildeten Einkopplungsoptik **37.4** aus der optischen Achse **65** weit ausgelenkten Strahl **LDB** (strichpunktiert). Ein analog zu den Spiegeln **45.1** bis **45.3** ausgebildeter Spiegel **45.4,** dessen reflektierende Oberfläche **69** parallel zur optischen Achse **65** angeordnet ist, lenkt den Strahl **LDB** hier so auf die Kornea **24.4,** dass der Strahl **LDB** näherungsweise senkrecht auf die periphere Kornea **24.4** auftrifft, so dass auch periphere Dickenmessungen der Kornea **24.4** ermöglicht werden. Der Messstrahl **SDB,** der nur leicht durch die Strahlablenkeinheit **67** aus der optischen Achse **65** ausgelenkt wird, trifft nicht auf den Spiegel **45.4** auf. Es ist nicht nötig, dass der Strahl **SDB** auf den Spiegel **45.4** auftrifft, weil der Strahl **SDB** noch genügend nahe an der zentralen Stelle **70** (bis ca. 1 mm) der Kornea **24.4** auftrifft, so dass die Abweichung vom senkrechten Einfall noch genügend klein ist, um ein Interferenzsignal zu detektieren. Erst für periphere Bereiche **71** der Kornea **24.4,** bei denen deren Krümmung sich bereits bemerkbar macht, muss diese Oberflächenkrümmung durch ein "Abknicken" des Strahls **LDB** mit dem Spiegel **45.4** korrigiert werden.

**Figur 6** zeigt eine beispielsweise Anordnung eines Messarms **73,** welcher anstelle der Messarme **31, 49a** und **54** verwendet werden kann. Der Messarm **73** weist eine polarisationserhaltende Faser **74** auf, welche mit einem Stecker **75** für diese polarisations erhaltende Faser **74** mit einer zu einem der Faserkoppler **29.1, 29.2** oder **53b** führenden Faser **76** verbunden ist. Es kann auch ein Stecker für nicht polarisationserhaltende Fasern verwendet werden. Im Gegensatz zur "normalen" Monomodefaser **76** kann die Lage der polarisationserhaltenden Faser **74** geändert werden, ohne dass dies Auswirkungen auf das erzeugte Interferenzsignal hat. Durch eine einstellbare Winkellage des Steckers **75** ist es möglich, die Polarisationsrichtung des aus dem Faserende **77** der Faser **74** austretenden Freiraummessstrahls **79** an die Polarisationsrichtung der Strahlung im Referenzarm 30, **49b bzw. 56b** anzupassen. Infolge der in **Figur 6** skizzierten Ausgestaltung des Messarms **73** kann auf die in den **Figuren 2** bis **4** und **9** in den dortigen Messarmen **31, 49a** bzw. **54** notwendigen Polarisationskontroller **33.1, 33.2** bzw. **33.3** verzichtet werden. Der Messarm und damit die gesamten Messeinrichtungen können durch den Wegfall der Polarisationskontroller **33.1, 33.2** bzw. **33.3** kleiner und billiger gebaut werden.

**Figur 7** zeigt ein Flussdiagramm, welches ein Verfahren bei der Abtragung für LASIK [Laser-assisted in situ Keratomileusis] darstellt. Das Ziel der LASIK sowie auch der anderen "refraktiven Operationen" wie z.B. PRK oder LASEK ist, Fehlsichtigkeiten, wie Kurz- oder Weitsichtigkeit, derart zu korrigieren, dass auf eine Brille bzw. Kontaktlinsen verzichtet werden kann. Es zeigt, wie und wann die Homhautdickenmessungen benutzt werden, um die "LASIK-Operation" zu steuern. Dieses Flussdiagramm gilt sowohl für myope als auch hyperope LASIK. Der einzige Unterschied zwischen den Verfahren für die myope und hyperope LASIK besteht darin, dass für die Berechnung des Abtragungsdurchmessers als Funktion der Dioptrienänderung eine unterschiedliche Formel benutzt wird. LASIK ist eine refraktive Operation, bei der mit Hilfe eines Mikrokeratoms ein Kornealappebn (Flap) von ca. 150 $\mu$m Dicke geschnitten wird. Dieser Lappen bleibt auf der einen Seite mit der Restkornea verbunden. Dieser Lappen wird dann umgeklappt. Auf das so freigelegte Stromä der Kornea werden dann Laserpulse geschossen, bis die gewünschte Abtragung erreicht ist. Nach Abschluss der Ablation wird der Kornealappen wieder zurückgeklappt und am ursprünglichen Ort plaziert.

In einem ersten Verfahrensschritt wird mit einer der oben beschriebenen Messeinrichtungen **1.1, 1.2** oder **1.3** die Dicke DMF der Kornea mit Flap bestimmt. Die Wegvariationslänge $\Delta z$ des Weglängenvariatoren **32.1, 32.2** bzw. **32.3** muss mindestens so groß sein, wie die auszumessende Dicke der Kornea. Interferenz tritt immer dann ein, wenn die Weglängen im Referenz- und im Messarm gleich lang sind. Da die Weglänge im Referenzarm mit den Weglängenvariatoren **32.1, 32.2 bzw. 32.3** geändert wird, wird nach Auswertung der mit dem Detektor **40** bzw. mit den beiden Detektoren **47a** und **47b** bzw. **63a** und **63b** detektierten Signale durch die betreffende Auswerteeinheit **11: 41,50.1,** sowie **50.2** jeweils ein Interferenzsignal festgestellt, wenn die Weglängen im Messarm bis zur Vorderseite der Komea sowie bis zu deren Rückseite gleich groß sind wie die Weglängen im Referenzarm. Die beiden Interferenzen ergeben dann mit einer Differenzbildung die Dicke DMF der Kornea **24.1** mit Flap. Diese Dikke DMF wird in einem zur Auswerteeinheit gehörenden Datenspeicher abgespeichert.

In einem nächsten Verfahrensschritt wird der Flap aufgeschnitten und weggeklappt Es wird nun eine erneute Dickenmessung DOF ohne Flap, analog zur obigen, Beschreibung, durchgeführt. Der hierbei ermittelte Dickenwert DOF wird ebenfalls abgespeichert. Von der Auswerteeinheit wird nun eine kritische Dicke KD unter Beachtung der Korneadicke mit und ohne Flap DMF und DOF sowie eventuell weiteren Patienten-spezifischen Parametern ermittelt. Die kritische Dicke KD ist ein Dikkenwert der Kornea, der u.a. aus Stabilitätsgründen nicht unterschritten werden darf. Es wird nun ein erster Laserpuls zur ersten Teilablation der Komea vorgenommen und anschließend die momentane Dicke MD der Komea mit dem oben erläuterten Messverfahren ermittelt. Eine Differenz zwischen Dickenwert DOF der Komea ohne Flap und diesem momentanen Dickenwert MD ergibt einen Dickenabtragungswert DAB pro Laserpuls. Da nun der Dickenabtragungswert DAB pro Laserpuls bekannt ist, kann einwandfrei ermittelt werden, wieviel Laserpulse zur Abtragung der Kornea für eine vorgegebene Korneärestdicke DREST notwendig sind. Die Anzahl notwendiger Laserpulse wird derart bestimmt, dass eine vorgegebene kritische Komeadicke KD nicht unterschritten wird. Vorgängig ist eine Dickenmessung mit Flap vorgenommen worden. Eine kritische Restdicke der Kornea kann von deren Ausgangsdicke abhängig sein. Bevorzugt wird man diese kritische Restdicke KD gemäß nachstehender Formel ermitteln:

$$KD = a \cdot DMF - b,$$

wobei DMF die Ausgangsdicke der Komea (d. h. vor der ersten Intervention an der Kornea) ist. Die Größen a und b werden vom Anmelder ermittelt. Als gegebenenfalls zu korrigierende Standardwerte sind a = 0,58 und b = 30 $\mu$m.

Anstatt den Flap aufzuschneiden und wegzuklappen, kann auch das Epithel weggeschabt werden. In diesem Fall wird einmal die Dicke der Komea mit dem Epithel und einmal nach Entfernen des Epithels gemessen. In den angeführten Verfahrensablauf wird dann der Wert DMF durch den Dickenwert mit Epithel DME und DOF durch denjenigen ohne Epithel DOE ersetzt. Figur 8 zeigt das hierzu gehörende Flussdiagramm, welches ein Verfahren bei der Abtragung für PRK [**p**hoto **r**efraktive **K**eratektometrie) darstellt. Es zeigt, wie und wann die Homhautdickenmessungen benutzt werden, um die PRK-Operation zu steuern. Dieses Flussdiagramm gilt sowohl für myope - als auch hyperope Korrekturen. Der einzige Unterschied zwischen dem Verfahren für die myope und hyperope PRK besteht darin, dass für die Berechnung des Abtragungsdurchmessers als Funktion der Dioptrienänderung eine unterschiedliche Formel benutzt werden muss.

**Figur 9** zeigt eine weitere Ausführungsvariante. Es wird hier eine periphere Koronadickenmessung oder Skleradickenmessung vorgenommen. Der Messstrahl und der Behandlungsstrahl verlaufen nun nicht mehr miteinander. Im Gegensatz zu der in **Figur 2** skizzierten Vorrichtung **1.1** wird hier die Messstrahlung mit einem Faserkoppler **85** in mehrere Teilstrahlungen aufgeteilt, wobei die Teilstrahlungen in Strahlungsleitern **86a, 86b,** ... mit bevorzugt einer ringförmigen Anordnung um einen Strahl **87** eines Behandlungslasers **89** herum angeordnet sind. Mit dieser Anordnung können periphere Homhautdickenmessungen vorgenommen werden. In Figur 9 sind der Übersichtlichkeit wegen nur zwei aus den beiden Strahlungsleitern **86a** und **86b** austretende Teilstrahlen **88a** und **88b** gezeichnet. Die aus den Fasern austretenden Messstrahlen **88a, 88b,** ... werden durch eine Optik **90** auf die Komea **24.6** oder die Sklera fokussiert. Der Durchmesser der ringförmigen Anordnung kann manuell oder automatisiert verändert werden, so dass die Komeadicke an verschiedenen konzentrischen Ringen mit unterschiedlichem Durchmesser gemessen werden kann. Das Interferenzsignal wird hier mit einem Detektor **94** detektiert und mit einer Signalverarbeitungseinheit **50.3** verarbeitet.

Eine Selektion unterschiedlicher Messorte kann durch eine entsprechende Ausbildung des Faserkopplers **85** erreicht werden. Dieser Faserkoppler **85** kann beispielsweise als faseroptischer Schalter ausgebildet sein, der in kurzer zeitlicher Abfolge die Strahlung von einem Strahlungsleiter in einen anderen Strahlungsleiter umschaltet. Somit sind die Interferenzsignale verschiedener Messorte zeitlich voneinander getrennt und können dadurch dem Messort zugeordnet werden. Die Schaltzeiten liegen typischerweise im Millisekundenbereich.

Anstelle des oben erwähnten Faserkopplers **85** kann auch ein wellenlängenselektiver Faserkoppler verwendet werden. Hierbei wird eine breitbandige Strahlung der Strahlungsquelle **26.3** im Frequenzraum auf mehrere Strahlungsleiter aufgeteilt. Die in den einzelnen Strahlungsleitern geführten Strahlungen sollten sich im Frequenzraum möglichst nicht überlappen. Auf der Detektorseite wird dann ein weiterer (nicht dargestellter) wellenlängenselektiver Faserkoppler verwendet, der die Strahlung auf eine den Teilfrequenzen entsprechende Anzahl Detektoren führt. Jedem Messort ist dann ein Detektor zugeordnet.

Figur **10** zeigt ebenfalls ein Ausführungsbeispiel zur peripheren Korneadickenmessung oder Skleradickenmessung, wobei auch hier ein Bündel Messstrahlen **91** nicht in den Strahlengang **92** eines Behandlungslasers **93** eingekoppelt wird. Analog zur Ausführung in **Figur 9** wird auch hier eine von einer Strahlungsquelle **26.4** kommende Messstrahlung **95** mit einem Strahlteiler **96** in einen Messarm und einen Referenzarm **97a** und **97b** aufgeteilt. Im Referenzarm **97b** wird, wie bereits oben erläutert, eine Referenzstrahlung **99** zu einem Weglängenvariator **100** geführt, mit einer optischen Weglängenvariation beaufschlagt und zurückreflektiert. Eine Messstrahlung **101** wird im Messarm **97a** mit einem Kollimationsarray **102** in mehrere Messstrahlen **91** aufgeteilt, wobei dann jeder Teilstrahl in einen Leiter eines Leiterbündels **103** eingekoppelt wird. Die Leiter des Bündels **103** sind in einer vorgegebenen Konfiguration vor einer auszumessenden Kornea **24.7** angeordnet. Die Leiter können kreisringförmig konfiguriert werden. Die von der Vorder- und Rückseite der Kornea **24.7** reflektierte Strahlung gelangt wieder in die einzelnen Leiter des Bündels **103** und wird durch das Kollimatorarray **102** in eine "Freiraumstrahlung" geformt, welche mit der vom Weglängenvariator reflektierte Strahlung interferiert. Die interferierende Strahlung wird mit einem ein- oder zweidimensionalen Detektorarray **105** detektiert und analog zu den oben gemachten Ausführungen mit einer parallelen Verstärkerschaltung, einer parallelen Filterschaltung, eventuell einer parallelen Gleichrichterschaltung und eventuell einer parallelen Enveloppenbildungsschaltung verarbeitet. Diese gesamte parallele Schaltung von der Photodetektion bis zur Enveloppenbildung kann beispielsweise auf einem CMOS-Chip integriert werden.

Die oben beschriebene Erfindung ermöglicht es vor, während und nach der Epithelentfernung oder vor, während und nach dem Aufschneiden eines Kornealappens die Korneadicke an einer oder mehreren Stellen der Kornea in Echtzeit zu messen. Die Erfindung ermöglicht es auch, vor, während und nach der photorefraktiven Korneabehandlung die Korneadicke an einer oder mehreren Stellen der Kornea zu messen. Die Erfindung kann zudem die Messdaten liefern, die nötig sind, um die Korneatopographie, die Krümmungsradien auf der Korneaoberfläche und die Korneaelevation zu bestimmen. Die erfindungsgemäße Vorrichtung kann bei verschiedenen chirurgischen Techniken verwendet werden (PRK, PTK, LASIK, LASEK). Die Vorrichtung kann einerseits verwendet werden, um den Behandlungslaser so zu steuern, dass eine vorher vorgegebene (eventuell patientenspezifische) kritische Korneadicke bei der Korneaabtragung nicht unterschritten wird. Vor allem bei der sogenannten LASIK-Technik kommt es vor, dass diese kritische Restdicke unterschritten wird. Ein Unterschreiten dieser kritischen Restdicke kann zu schweren Komplikationen führen. Der Erfindungsgegenstand wird bevorzugt mit einer Software und einer Verbindung zum Behandlungslaser derart ausgerüstet, dass aufgrund der Echtzeit-Messung der Korneadicke der Lasereingriff selbsttätig steuerbar ist. Bei Bedarf können somit Laserparameter so abgeändert werden, dass dennoch eine vorgegebene Dioptrienänderung durchgeführt werden kann, ohne die kritische Korneadicke zu unterschreiten. Dies wird erreicht, indem der Durchmesser der Ablationszone bei Bedarf während der Behandlung verkleinert wird. Durch die Verkleinerung des Durchmessers der Ablationszone wird die vorgegebene Dioptrienänderung schon bei einer geringeren Ablationstiefe erreicht, so dass die Gefahr, die kritische Korneadicke KD zu unterschreiten, gebannt ist. Für die Myopiekorrektur ist der Zusammenhang zwischen Dioptrienänderung, Durchmesser der Ablationszone und der zentralen Ablationstiefe näherungsweise durch folgende Formel, gemäß Cornea, Vol. III, Surgery of the Cornea and Conjunctiva, Jay H. Krachmer, Mark J. Mannis, Edward J. Holland; Mosby, 1997, gegeben:

$$t_o = -(S^2 D) / 3,$$

wobei $t_o$ die maximale Abtragungstiefe auf der optischen Achse in µm, **S** der Durchmesser der Abtragungszone in mm und D die Dioptrienänderung in dpt bedeutet. Bei der Hyperopiekorrektur gilt näherungsweise dieselbe Formel, jedoch bedeutet $t_o$ in diesem Fall die maximale Abtragungstiefe in der Peripherie der Kornea, wobei vorausgesetzt ist, dass im Zentrum der Kornea keine Abtragung stattfindet. Genauere und allgemeinere Formeln für die Dioptrieänderung als Funktion der Abtragung befinden sich ebenfalls in der oben genannten Literatur.

Der Messstrahl kann an verschiedenen Orten der als Behandlungslasergerät ausgebildeten Vorrichtung eingekoppelt werden. Dabei sind die Orte mit Umlenkspiegeln zu bevorzugen. Eine Einkopplung an diesen Orten ist einfach, da lediglich der Umklenkspiegel durch einen entsprechend für die Wellenlängen der verwendeten Strahlungen ausgebildeten Strahlteiler zu ersetzen ist.

Der Strahlenteiler (oder Teilerspiegel) ist Bestandteil der Einkopplungsoptik **37.1** bis **5.** Die Einkopplungsoptik **37.1** bis **5** besteht neben diesem Strahlenteiler aus einer Singlemodefaser, einem Faserstecker und einer Linse (oder einem Linsensystem), Dabei ist die Reihenfolge dieser Komponenten in Richtung vom Faserkoppler zum Auge folgendermassen: Singelmodefaser **35**bzw. **74,** Faserstecker **36.1 b** bzw. **77,** Linse (oder Linsensystem), Strahlenteiler (oder Teilerspiegel). Faserstecker und Linse (oder Linsensystem) bilden dabei die Strahlformungseinheit **6.** Die Strahlformungseinheit **6** hat die Aufgabe, den Messstrahl so zu formen, dass sein Strahlquerschnitt einerseits die von dem Behandlungsgerät vorgegebenen Öffnungsdurchmesser möglichst nicht überschreitet und andererseits (in Kombination mit der vom Behandlungsgerät vorgegebenen Optik) auf der Kornea fokussiert wird.

Die Verwendung hier von zwei Linsen bei der Strahlformungseinheit 6 ist nicht zwingend; es können statt dessen auch Scannerspiegel verwendet werden.

Der Messstrahl kann nicht nur an denjenigen Orten des Behandlungslasergerätes, an denen ein Umlenkspiegel vorliegt, eingekoppelt werden. Es kann auch im Strahlengang einer Videokamera oder eines Fixationslasers, welche beide in der Regel in einem Behandlungsgerät vorhanden sind eingekoppelt werden. In diesem Fall ist aber ein zusätzlicher Strahlenteiler nötig. Dieser Strahlenteiler teilt dann entweder die Strahlung des Messstrahls und die Strahlung des Fixationslasers. Da die Wellenlänge des Messstrahls von 1300 nm sich sowohl stark von der normalerweise für die Videokamera benutzen Strahlungswellenlänge von ca. 850 nm als auch stark von der normalerweise benutzten Wellenlänge des Fixationslasers von ca. 650 nm unterscheidet, ist die Beschichtung des benötigten Strahlungsteilers einfach herstellbar.

Die Messeinrichtung **1.1, 1.2** bzw. **1.3** können zusammen mit einem Stereomikroskop - in der Regel mit einem großen Arbeitsabstand von ca. 30 cm - verwendet werden. Der Messstrahl eines oben und in Varianten auch noch unten beschriebenen Kurzkohärenzreflektometers kann nun in einen der beiden Strahlengänge des Stereomikroskops eingeblendet werden. Soll zudem eine Videokamera vorgesehen werden, kann mit einem Strahlteiler gearbeitet werden. Nachteilig ist bei einer Einkopplung des Messstrahls in den Strahlengang eines Stereomikroskops ein nicht mehr senkrechtes Auftreffen des Messstrahls auf der Kornea. Zur Erzeugung eines Stereobeobachtungseffektes müssen nämlich die beiden Beobachtungsstrahlen zueinander geneigt sein. Ein Messstrahl der Messeinrichtung kann auch in die optische Achse eines Stereomikroskops geführt und dort mit einem kleinen Umlenkspiegel (mittig zwischen den beiden Beobachtungsstrahlen), welcher die beiden Stereobeobachtungsstrahlengänge so wenig wie möglich stören sollte, durch das Objektiv auf eine zu behandelnde bzw. auszumessende Kornea geführt werden. In diesem Fall muss die numerische Apertur des Stereomikroskops genügend groß sein, um die spekulär reflektierende Messstrahlung aufzunehmen. Anderenfalls muss die Empfindlichkeit der Photodetektoren **40, 47a, 47b** bzw. **63a** und **63b** der Messeinrichtung **1.1, 1.2** bzw. **1.3** genügend hoch sein, um die in den gesamten Halbraum zurückgestreute Messstrahlung zu detektieren.

Bei einer weiteren Verwendungsvariante der Messeinrichtungen **1.1, 1.2** bzw. **1.3** zusammen mit einem Stereomikroskop fällt die optische Achse des Stereomikroskops und die Achse des Behandlungsstrahlengangs zusammen. Für die Messstrahlung der Messeinrichtungen **1.1, 1.2** bzw. **1.3** ist dann ein Strahlteiler in jedem Stereobeobachtungsgang, d. h. außerhalb des Behandlungsstrahlengangs angeordnet. Im Gegensatz zur vorgängigen Ausführungsvariante sind hier Bedingungen an die numerische Apertur des Stereomikroskops und die Empfindlichkeit der Photodetektoren weniger streng. Wird auf eine Stereobetrachtung verzichtet, so können die einzelnen "Stereostrahlengänge" für unterschiedliche Verwendungen benutzt werden. Es kann beispielsweise einer der beiden Strahlengänge für eine Videobetrachtung verwendet werden.

Eine weitere Ausführungsvariante ist in **Figur 11** dargestellt, wobei hier eine Messeinrichtung **1.6** gegenüber den Messeinrichtungen **1.1, 1.2, 1.3** und **1.4** in den **Figuren 2, 3, 4** und **9** leicht variiert ist. Die Messeinrichtung arbeitet auch hier mit einem Stereomikroskop **133** zusammen. Die freie Messstrahlung **135** wird jedoch hier mit einem ersten Strahlteiler **137a** in den einen Strahlengang **139a** des Mikroskops **133** eingekoppelt und mit einem weiteren Strahlteiler **137b** nach Reflexion an Vorder- und Rückseite der auszumessenden Kornea **24.8** aus dem anderen Strahlengang **139b** ausgekoppelt. Die Messeinrichtung **1.6** hat hier zwei Faserkoppler **140a** und **140b** und zwei Detektoren **142a** und **142b.** Mit dem Faserkoppler **140a** wird die von einer Strahlungsquelle **26.5** kommende Strahlung in einen Strahlungsleiter **143** und in einen Referenzarm **144** mit einem Weglängenvariator **32.4,** wie bereits oben erläutert, aufgeteilt. Die im Strahlungsleiter **143** geführte Strahlung wird, analog zu den oben beschriebenen Ausführungsvarianten mit einer Auskopplungseinheit (Kollimatoreinheit) **37.4** zum parallelen Freiraumstrahl **135** geformt. Der von der Kornea **24.8** reflektierte und vom Strahlteiler **137b** umgelenkte Freiraumstrahl **147** wird mit einer Einkopplungseinheit (Kollimatoreinheit) **37.5** in einem Strahlungsleiter **150** eingekoppelt. Der Strahlungsleiter **150** ist mit dem Faserkoppler **140b** verbunden. Im Faserkoppler **140b** interferieren dann der vom Weglängenvariator **32.4** reflektierte Strahlungsteil, der über einen Strahlungsleiter **151** vom Faserkoppler **140a** zum Faserkoppler **140b** geleitet wird, mit dem über den Strahlungsleiter **150** kommenden, an der Komea **24.8** reflektierten Strahlungsteil. Die optische Weglänge vom Faserkoppler **140a** über den linken und rechten Strahlengang **139a** und **139b** des Mikroskops **133** bis zum Faserkoppler **140b** muss mit einer durch die Kohärenzlänge der Strahlungsquelle **26.5** gegebenen Toleranz gleich lang sein wie diejenige vom Faserkoppler **140a** zum Weglängenvariator **32.4** und zurück bis zum Faserkoppler **140b.** Im Faserkoppler **140b** entsteht dann bei einer derartigen Übereinstimmung der Weglängen eine Interferenz der zwei durch den Faserkoppler **140a** getrennten Strahlungen. Die Interferenz wird von den beiden Detektoren detektiert, wobei auch ein einziger Detektor ausreichend ist, wenn der Signalrauschanteil gering ist.

Unabhängig von Ort und Art einer Einkopplung der Messstrahlung in den Strahlengang eines Mikroskops können auch ermittelte Messwerte und andere Informationen, wie die momentane Restdicke der Komea oder die noch fehlende Distanz bis zur kritischen Dicke $d_{krit}$ der Komea bildtechnisch eingekoppelt werden. Diese Daten können als Zahlen und/oder Buchstaben und/ oder unterschiedliche Farbkennzeichnungen über einen (nicht dargestellten) Strahlungsteiler in das Okular des Mikroskops eingeblendet werden.

Es kann auch eine Taste oder ein Fußpedal vorgesehen werden, um einen Zeitpunkt festzulegen, an dem der zum betreffenden Zeitpunkt anstehende Messwert hervorhebend gekennzeichnet, mit dieser Hervorhebung in einem Speicher abgespeichert und/oder auf einer Anzeige oder einem Monitor mit dieser Hervorhebung dargestellt wird. Besondere Zeitpunkte können beispielsweise sein:

- der Zeitpunkt unmittelbar vor oder nach dem Aufsetzen eines Saugrings eines Mikrokeratoms,
- der Zeitpunkt unmittelbar vor oder nach dem Schneiden eines kornealen Flaps,
- der Zeitpunkt unmittelbar vor oder nach dem Wegkratzen des Korneaepitheliums,
- der Zeitpunkt unmittelbar vor oder nach dem Spülen der Kornea und/oder der Sklera,
- der Zeitpunkt unmittelbar vor dem ersten bzw. dem letzten Laserpuls oder
- der Zeitpunkt unmittelbar vor oder nach dem Zurückklappen eines kornealen Flaps.

Mit der oben erläuterten Messung kann eine Verdünnung der Kornea gemessen und mit einer geplanten Verdünnung graphisch und rechnerisch verglichen werden. Es können auch verdünnungsrelevante Faktoren wie z. B. Wasserverdampfung aus der Kornea und/ oder verdickungsrelevante Faktoren, wie Schwellungen durch einen chirurgischen Eingriff ermittelt werden. Die pro Zeiteinheit ermittelte Wasserverdampfung wird dann dazu benutzt, einen chirurgischen oder therapeutischen Laserstrahl in Echtzeit zu steuern. Korneadickenwerte, welche eine Funktion der Luftfeuchtigkeit und der Lufttemperatur sind, wird man abspeichern und bei der Ablation heranziehen, um Umgebungsbedingungen im Operationsraum auszugleichen. Es können auch typische Korneadickenwerte betreffend Alter, Geschlecht und Zusammensetzung der Kornea sowie deren Temperaturverhalten abgespeichert und zur betreffenden Behandlung dann abgerufen werden.

Durch die oben beschriebenen Verfahren konnte zudem ermittelt werden, dass eine patientenunabhängige Abtragungsrate der Kornea für zufriedenstellende Ergebnisse nicht ausreichend ist. Es kann auch nicht, wie bisher üblich, vorausgesetzt werden, dass der Abtragungslaser während der gesamten Abtragung pro Laserpuls eine konstant dicke Korneaschicht abträgt. Eine Eichung einer Abtragungsrate nur einmal in einem vorgegebenen Zeitrahmen (bisher meist ein Tag) ist somit nicht ausreichend. Es kann sich zudem der Wassergehalt und die Verdünnung der Kornea infolge Verdampfung des in der Kornea enthaltenen Wassers patientenabhängig ändern.

Um die Korneadickenmessung mit der beabsichtigten Abtragung des Lasers zu korrelieren, genügt es demnach nicht, nur die Korneadicke zu einem vorgegebenen Zeitpunkt zu kennen. Es ist nämlich zudem für eine optimale Behandlung notwendig, auch den Zeitpunkt des vollzogenen Flapschneidens oder den Zeitpunkt der vollzogenen Epithelabkratzung und dann die Korneaverdünnung pro Zeiteinheit infolge Verdampfung zu kennen. Auch kann sich die Korneadicke nach einer Behandlung aus verschiedenen Gründen noch ändern.

Die Laserabtragungspulse können nun aufgrund von eingegebenen Erfahrungswerten automatisch gesteuert werden. Diese Erfahrungswerte können beispielsweise vom Alter, Geschlecht und der Krankengeschichte abhängen. Die Erfahrungswerte können aber auch mit dem oben beschriebenen Messverfahren vorerst ohne Ablation ermittelt werden.

Die oben beschriebenen Korneadickenmessungen können noch mit einer Distanzmessung und/ oder einer "Ausrichtungsmessung" ergänzt werden, um die gewünschte Abtragung auch dann exakt auszuführen, wenn das Abtragungsverhalten des Lasers abhängig von der Position des Patientenauges wäre. Je nach numerischer Apertur des Laserstrahls und der Operationstechnik ist dieser Einfluss vernachlässigbar oder muss berücksichtigt werden. Wird eine Distanzänderung zwischen einem Referenzpunkt und der Patientenkornea berucksichtigt, kann die Abtragungsrate besser gesteuert werden. Jeder Laserstrahl hat nämlich eine gewisse Divergenz, welche bei einer Veränderung der Position des Patientenauges in der Ausbreitungsrichtung des Strahles einen veränderten Strahldurchmesser auf dem Auge hervorruft.

Die numerische Apertur des Messstrahls gibt eine maximale Winkeltoleranz vor, mit der das Auge (oder die Kornea) des Patienten von einer optimal ausgerichteten Lage ohne Verlust des Messsignals abweichen darf. Fixiert nämlich der Patient nicht exakt einen Fixationslaserpunkt, so trifft der Messstrahl nicht mehr senkrecht auf die beiden Korneaflächen. Der Messstrahl wird nicht mehr in sich selbst rückreflektiert, wodurch kein Messsignal detektiert werden kann. Es erfolgt dann ein automatischer Unterbruch der Korneaablation, da sich ansonsten Ablationsfehler ergeben würden.

Würde zur Überwachung lediglich eine CCD-Kamera verwendet werden, so könnte nur ermittelt werden, wie weit die Mitte der Pupille von der Abtragungslaserachse entfernt ist. Bei dieser Überwachungsmethode kann aber nicht zwischen einer lateralen Verschiebung der Kornea und einer mangelhaften winkelmäßigen Ausrichtung unterschieden werden.

Der die refraktiven Daten ebenfalls beeinflussende Wassergehalt der Kornea lässt sich durch eine Messung mit zwei unterschiedlichen Messwellenlängen bestimmen, wobei eine Wellenlänge z. B. im Bereich zwischen 450 nm und 1300 nm und die andere Wellenlänge nahe an einem Absorptionsmaximum von Wasser liegt. Das Absorptionsmaximum von Wasser liegt bei einer Wellenlänge vom 1800 nm und 400 nm. Bei einer Korneaverdünnung infolge einer Wasserverdampfung ändert sich die von der einen Wellenlänge gemessene Korneadicke anders als die von der anderen Wellenlänge. Aus diesen unterschiedlichen Werten kann auf den Wassergehalt geschlossen werden.

Eine Anordnung zur Ermittlung des Wassergehaltes zeigen die **Figuren 12** und **13.** Der in **Figur 12** dargestellte Aufbau hat zwei Strahlungsquellen **26.6** und **26,7,** welche analog zu obigen Ausführungen Strahlungen mit unterschiedlicher Wellenlänge $\lambda_1$ und $\lambda_2$ emittieren. Die beiden Strahlungen mit unterschiedlicher Wellenlänge werden in einem einzigen Strahlungsleiter zu einem Faserkoppler **155** geführt. Aus dem Faserkoppler **155** führt ein Wellenleiter **156** über einen Polarisationskontroller **157.1** und eine optische Einheit (Behandlungsgerät) **159.1** zur Kornea **24.9.** Vom Faserkoppler **155** führt ein weiterer Strahlungsleiter **160** zu einem Weglängenvariator **32.5.** Das Interferenzsignal wird vom Faserkoppler **155** über einen Strahlungsleiter **161** und einer Auftrennung auf die unterschiedlichen Wellenlängen auf zwei Detektoren **162a** und **162b** geführt. Die Signale der beiden Detektoren **162a** und **162b** werden in einer Auswerteschaltung **163.1** zur Ermittlung des Wassergehaltes ausgewertet. Die jeweiligen Resultate können dann auf einem Bildschirm **164.1** graphisch dargestellt werden.

Eine hierzu analoge Anordnungsvariante ist in **Figur 13** dargestellt. Auch hier werden zwei Strahlungsquellen **26.8** und **26.9,** welche Strahlungen mit unterschiedlicher Wellenlänge $\lambda_1$ und $\lambda_2$ aussenden, zwei Detektoren **162c** und **162d** für die Interferenzsignale der unterschiedlichen Wellenlängen $\lambda_1$ und $\lambda_2$, ein Weglängenvariator **32.5,** eine optische Einheit **159.2,** eine Auswerteschaltung **163.2,** ein Polarisationskontroller **157.2** und ein Bildschirm **164.2** sowie zwei Faserkoppler verwendet.

Über einen Strahlteiler können die oben beschriebenen Korneadickenmessgeräte bzw. Distanzmessgeräte [Distanzmessung von einem Referenzpunkt zur Korneavorderseite) zu einem neuartigen Laser-Keratom integriert werden. Neben den bereits oben angeführten Vorteilen ist dann zusätzlich eine Messung der Flapdicke mit Mikrometergenauigkeit möglich. Mit diesem Laser-Keraton wird mit kurzen Pulsen bevorzugt im Femtosekundenbereich ein kornealer Flap geschnitten. An das Flapschneiden schliesst dann die bereits oben beschriebene Abtragung der Komea an.

Bisher wurde der Flap mechanisch mit einem sogenannten Mikrokeratom unter Verwendung einer motorisierten Klinge geschnitten. Die bekannten Mikrokeratome hatten einen Saugring, mit dem sie auf die Augenoberfläche gepresst wurden, um ein Verrutschen während des Schneidvorgangs zu verhindern. Bei dem bekannten Verfahren traten oftmals Schneidfehler ("free caps", "button holes") auf, zudem entstand durch die mechanische Augenberührung ein zwar kurzzeitiger, aber doch unerwünschter Überdruck im Auge. Mit dem neuen Laser-Keratom werden diese Nachteile vermieden.

## Patentansprüche

1. Vorrichtung mit einer Laserstrahlungsquelle **(7, 89, 93)** zur Materialablation von der Oberfläche eines strahlungstechnisch transparenten oder diffusiven Gegenstands **(24.1 bis 24.10),** mit der eine Vielzahl hintereinander folgender Teilablationsvorgänge strahlentechnisch vornehmbar ist, vor und/oder nach jedem Teilablationsvorgang der relative Abstand des Teilablationsbereichs von einem Referenzwert zusammen mit der tatsächlichen Gegenstandsdicke Michelson-interferometrisch selbsttätig bestimmbar und lediglich sofern ein vorgegebener Abstandswert innerhalb einer Toleranz ermittelbar ist, entsprechend dem jeweils ermittelten Dikken-

wert ein nachfolgender Teilablationswert selbsttätig derart vorgebbar ist, dass nach Ablationsende ein vorgegebener Dickenverlauf oder ein zwei- bzw. ein dreidimensionales Dickenprofil des bearbeiteten Gegenstands **(24.1 bis 24.12)** erhaltbar ist, wobei die Vorrichtung eine Steuereinrichtung **(3),** eine von der Steuereinrichtung **(11)** gesteuerte Laserstrahlungsquelle **(7),** eine Messeinrichtung **(1, 1.1 bis 1.6)** mit einem Michelson-Interferometer und eine mit der Steuereinrichtung **(3)** signalmässig verbundene elektronische Auswerteeinheit **(41, 50.1, 50.2)** hat, welche derart ausgebildet ist, dass sie aus den mit der Messeinrichtung **(1, 1.1 bis 1.6)** ermittelten Messwerten den relativen Abstand des Teilablationsbereichs von einem Referenzwert zusammen mit der Gegenstandsdicke und aus letzteren der vorgegebene Dickenverlauf oder das zwei- bzw. dreidimensionale Dickenprofil des Gegenstands **(24.1 bis 24.10)** ermittelt und aufgrund des ermittelten Dikkenprofils die Laserstrahlungsquelle steuert, wobei diese nur freigegeben wird, sofern die Steuereinrichtung einen in einem vorgegebenen Toleranzbereich liegenden abgespeicherten Abstandswert und/oder insbesondere eine Neigung des Teilablationsbereichs innerhalb abgespeicherter Toleranzwerte ermittelt hat, **dadurch gekennzeichnet, dass** die Messeinrichtung **(1.1 bis 1.6)** mit dem Michelson-Interferometer eine Messstrahlungsquelle **(32.1 bis 32.4)** mit einer Zentrumswellenlänge hat, wobei die Zentrumswellenlänge in einer Bandbreite von ± 100 nm um eine Wellenlänge von 1'310 nm liegt, und im Referenzarm **(30; 49b; 56a, 56b, 32.3; 144)** eine Monomode-Faser **(35, 74, 76)** für diese Wellenlänge verwendet wird, damit die optische Weglänge in Luft im Messarm **(31; 49a; 54),** welche für die Durchführung der Messung notwendig ist, einwandfrei kompensierbar ist.

**2.** Vorrichtung nach Anspruch 1, **dadurch gekennzeichnet, dass** die Messeinrichtung **(1, 1.1 bis 1.6)** einen Weglängenvariator und die Auswerteeinheit wenigstens einen Speicher aufweist, wobei die Wegvariationslänge mindestens so gross ist, wie die auszumessende Dicke der der noch unbehandelten Kornea und im Speicher wenigstens ein Dickenwert **(DMF)** der unbehandelten Kornea als Differenz einer ersten Wegvariationslänge einer ersten auftretenden Interferenz betreffend Kornearückseite und einer zweiten Wegvariationslänge einer zweiten auftretenden Interferenz betreffend Korneavorderseite und wenigstens ein weiterer_Dickenwert **(DOF)** der Kornea mit in sie eingeschnittenem und weggeklapptem Kornealappen (Flap) abspeicherbar ist.

**3.** Vorrichtung nach Anspruch 2, **dadurch gekennzeichnet, dass** mit der Messeinrichtung zusätzlich ein Dickenwert der Kornea mit eingeschnittenem, aber noch nicht weggeklapptem Kornealappen (Flap) ermittelbar und im Speicher abspeicherbar ist.

**4.** Vorrichtung nach Anspruch 1, **dadurch gekennzeichnet, dass** die Messeinrichtung **(1, 1.1 bis 1.6)** einen Weglängenvariator und die Auswerteeinheit wenigstens einen Speicher aufweist, wobei die Wegvariationslänge mindestens so gross ist, wie die auszumessende Dicke der Kornea und im Speicher wenigstens ein Dikkenwert **(DME)** der Komea als Differenz einer ersten Wegvariationslänge einer ersten auftretenden Interferenz betreffend Kornearückseite und einer zweiten Wegvariationslänge einer zweiten auftretenden Interferenz betreffend Korneavorderseite und wenigstens ein weiterer Dickenwert **(DOE)** der Kornea mit weggeschabtem Epithel abspeicherbar ist.

**5.** Vorrichtung nach Anspruch 2 bis 4, **dadurch gekennzeichnet, dass** nach einer ersten Materialablation mit einem ersten Laserpuls auf die Kornea ohne Kornealappen bzw. ohne Epithel ein dritter Dikkenwert ermittelbar und im Speicher abspeicherbar ist, um einen Ablationsrichtwert **(DAB)** für jeden nachfolgenden Laserpuls zu erhalten und der jeweils ermittelbare dritte Dikkenwert **(MD)** mit einem im Speicher abspeicherbaren kritischen Dickenwert **(KD)** mit der Auswerteeinheit vergleichbar ist.

**6.** Vorrichtung nach einem der Ansprüche 1 bis 5, **gekennzeichnet durch** eine Strahlablenkeinheit, mit der, **durch** die Steuereinrichtung steuerbar, der Laserstrahl der Messstrahlungsquelle über die Gegenstandsoberfläche des Gegenstands ablenkbar ist.

**7.** Vorrichtung nach einem der Ansprüche 1 bis 5, **gekennzeichnet durch** eine Strahlablenkeinheit, mit der, **durch** die Steuereinrichtung steuerbar, der Laserstrahl der Messstrahlungsquelle über eine Augenkornea als Gegenstandsoberfläche linear ablenkbar ist.

**8.** Vorrichtung nach einem der Ansprüche 1 bis 5, **gekennzeichnet durch** eine Strahlablenkeinheit **(19, 67),** mit der, **durch** die Steuereinrichtung steuerbar, der Laserstrahl der Messstrahlungsquelle über die Gegenstandsoberfläche, flächig zur Ermittlung eines dreidimensionalen Dickenprofils des Gegenstands ablenkbar ist.

**9.** Vorrichtung nach einem der Ansprüche 1 bis 8, **dadurch gekennzeichnet, dass** das Michelson-Interferometer der Messeinrichtung **(1.4; 1.5)** einen Referenz- und einen Messarm hat, wobei der Messarm eine Mehrfach-Strahlteilereinheit **(85, 102)** hat, mit der der Messstrahl **(101)** in mehrere Messstrahlen aufspaltbar ist, und das Interferometer einen als Detektorarray **(105)** ausgebildeten Detektor für die Interferenzen zwischen Referenz- und Messstrahlung hat.

**10.** Vorrichtung nach einem der Ansprüche 1 bis 8, **dadurch gekennzeichnet, dass** das Michelson-Interferometer der Messeinrichtung **(1.4; 1.5)** einen Referenz- und einen Messarm hat, wobei der Messarm ein Kollimatorarray **(102)** hat, mit dem der Messstrahl **(101)** in mehrere Messstrahlen aufspaltbar ist, und das Interferometer einen als Detektorarray **(105)** ausgebildeten Detektor für die Interferenzen zwischen Referenz- und Messstrahlung hat.

**11.** Vorrichtung nach Anspruch 9 oder 10, **gekennzeichnet durch** ein Faserbündel **(86a, 86b; 103)** mit dem die **durch** die Mehrfach-Strahlerteileinheit **(85, 102)** oder **durch** das Kollimatorarray **(102)** der in mehrere Messstrahlen aufspaltbare Messstrahl **(101)** auf den Gegenstand zur Ermittlung des Dikkenverlaufs oder zur zwei- bzw. zur dreidimensionalen Profilermittlung führbar sind.

**12.** Vorrichtung nach einem der Ansprüche 1 bis 11, **dadurch gekennzeichnet, dass** die Strahlablenkeinheit derart ausgebildet ist, dass der Messstrahl des Michelson-Interferometers bis auf eine Winkeltoleranz in von der optischen Achse **(65)** abweichenden Positionen senkrecht auch auf eine nach aussen gewölbte Oberfläche des Gegenstands trifft.

**13.** Vorrichtung nach Anspruch 12, **dadurch gekennzeichnet, dass** die Strahlablenkeinheit als innen verspiegelter Hohlzylinder **(45.1 bis 45.5)** ausgebildet ist.

**14.** Vorrichtung nach Anspruch 12 oder 13, **dadurch gekennzeichnet, dass** die nach aussen gewölbte Oberfläche die Korneaoberfläche **(24.4)** ist, wodurch auch bei Patienten mit schlechter bzw. verminderter Fixationsfähigkeit und/oder unregelmässiger Korneaoberfläche die Korneadicke in einem möglichst weiten Bereich um das Korneazentrum herum messbar ist.

**15.** Vorrichtung nach einem der Ansprüche 1 bis 14, **gekennzeichnet durch** ein Mikroskop, dessen den Gegenstand **(24.8; 24.9; 24.10)** beobachtender Beobachtungsteilstrahlengang bzw. beobachtende Strahlengänge ein partieller Teil des Messarms der Messeinrichtung ist bzw. sind und eine Verkopplung von Beobachtungsteilstrahlengang und Teilstrahlengang des Messarms **durch** wenigstens einen Strahlteiler **(129a, 129b; 137a, 137b)** erfolgt.

## Claims

**1.** Apparatus having a laser radiation source **(7, 89, 93)** for material ablation of the surface of a radiation-transparent or radiation-diffusive object **(24.1 to 24.10),** with which a multiplicity of consecutive partial ablation operations by means of radiation may be performed, before and/or after each partial ablation operation the relative distance of the partial ablation region from a reference value together with the actual thickness of the object may automatically be determined by a Michelson interferometer, and only if a predefined distance value within a tolerance may be ascertained, in accordance with the thickness value respectively ascertained a subsequent partial ablation value may automatically be predefined in such a manner that, after the end of the ablation, a predefined thickness progression or a two-dimensional or a three-dimensional thickness profile of the treated object **(24.1 to 24.12)** may be obtained, wherein the apparatus has a control device **(3),** a laser radiation source **(7)** controlled by the control device **(11),** a measuring device **(1,1.1 to 1.6)** with a Michelson interferometer, and an electronic evaluation unit **(41, 50.1, 50.2)** which is in signal communication with the control device **(3)** and which is configured in such a manner that it ascertains from the measurements ascertained by the measuring device **(1, 1.1 to 1.6)** the relative distance of the partial ablation region from a reference value together with the thickness of the object and, from the latter, the predefined thickness progression or the two-dimensional or three-dimensional thickness profile of the object **(24.1 to 24.10)** and controls the laser radiation source on the basis of the thickness profile ascertained, the laser radiation source being enabled only if the control device has ascertained a stored distance value lying in a predefined tolerance range, and/or especially has determined an inclination of the partial ablation region within stored tolerance values, **characterised in that** the measuring device **(1.1 to 1.6)** with the Michelson interferometer has a measurement radiation source **(32.1 to 32.4)** with a centre wavelength, the centre wavelength lying around a wavelength of 1310 nm in a bandwidth of ± 100 nm, and a monomode fibre **(35, 74, 76)** being used for that wavelength in the reference arm **(30; 49b; 56a, 56b, 32.3; 144)** in order that the optical path length in air in the measuring arm **(31; 49a; 54),** which is required for carrying out the measurement, can be compensated for in a satisfactory manner.

**2.** Apparatus according to claim 1, **characterised in that** the measuring device **(1,1.1 to 1.6)** has a path length variator and the evaluation unit has at least one memory, wherein the path variation length is at least as great as the thickness of the as yet untreated cornea to be measured, and at least one thickness value **(DMF)** of the untreated cornea in the form of the difference between a first path variation length of a first occurring interference pertaining to the rear side of the cornea and a second path variation length of a second occurring interference pertaining to the front side of the cornea, and at least one further thickness value **(DOF)** of the cornea with a flap cut therein and folded away may be stored in the memory.

**3.** Apparatus according to claim 2, **characterised in that**, in addition, a thickness value of the cornea with a flap cut therein but not yet folded away may be ascertained by the measuring device and may be stored in the memory.

**4.** Apparatus according to claim 1, **characterised in that** the measuring device **(1, 1,1 to 1.6)** has a path length variator and the evaluation unit has at least one memory, wherein the path variation length is at least as great as the thickness of the cornea to be measured, and at least one thickness value **(DME)** of the cornea in the form of the difference between a first path variation length of a first occurring interference pertaining to the rear side of the cornea and a second path variation length of a second occurring interference pertaining to the front side of the cornea, and at least one further thickness value **(DOE)** of the cornea with the epithelium scraped away may be stored in the memory.

**5.** Apparatus according to claim 2 to 4, **characterised in that**, following a first material ablation with a first laser pulse, a third thickness value may be ascertained on the cornea without flap or without epithelium and may be stored in the memory in order to obtain an ablation guide value **(DAB)** for each successive laser pulse, and the respectively ascertainable third thickness value **(MD)** may be compared using the evaluation unit with a critical thickness value **(KD)** storable in the memory.

**6.** Apparatus according to any one of claims 1 to 5, **characterised by** a beam deflecting unit with which, in a manner controllable by the control device, the laser beam of the measurement radiation source may be deflected over the surface of the object.

**7.** Apparatus according to any one of claims 1 to 5, **characterised by** a beam deflecting unit with which, in a manner controllable by the control device, the laser beam of the measurement radiation source may be deflected linearly over an eye cornea forming the surface of the object.

**8.** Apparatus according to any one of claims 1 to 5, **characterised by** a beam deflecting unit **(19, 67)** with which, in a manner controllable by the control device, the laser beam of the measurement radiation source may be deflected over the surface of the object in a planar manner in order to ascertain a three-dimensional thickness profile of the object.

**9.** Apparatus according to any one of claims 1 to 8, **characterised in that** the Michelson interferometer of the measuring device **(1.4; 1.5)** has a reference arm and a measuring arm, wherein the measuring arm has a multiple beam splitting unit **(85, 102)** with which the measuring beam **(101)** may be split into a plurality of measuring beams, and the interferometer has a detector configured as a detector array **(105)** for the interferences between reference radiation and measurement radiation.

**10.** Apparatus according to any one of claims 1 to 8, **characterised In that** the Michelson interferometer of the measuring device **(1.4; 1.5)** has a reference arm and a measuring arm, wherein the measuring arm has a collimator array **(102)** with which the measuring beam **(101)** may be split into a plurality of measuring beams, and the interferometer has a detector configured as a detector array **(105)** for the interferences between reference radiation and measurement radiation.

**11.** Apparatus according to claim 9 or 10, **characterised by** a fibre bundle **(86a, 86b; 103)** with which the measuring beam **(101),** which is splittable into a plurality of measuring beams by the multiple beam splitting unit **(85, 102)** or by the collimator array **(102),** may be guided onto the object in order to ascertain the thickness progression or to ascertain the two-dimensional or the three-dimensional profile.

**12.** Apparatus according to any one of claims 1 to 11, **characterised in that** the beam deflecting unit is configured in such a manner that the measuring beam of the Michelson interferometer impinges perpendicularly also on an outwardly curved surface of the object except for an angle tolerance in positions deviating from the optical axis **(65).**

**13.** Apparatus according to claim 12, **characterised in that** the beam deflecting unit is configured as an internally reflectively coated hollow cylinder **(45.1 to 45.5).**

**14.** Apparatus according to claim 12 or 13, **characterised in that** the outwardly curved surface is the cornea surface **(24.4),** whereby the thickness of the cornea may be measured in as wide a region as possible around the centre of the cornea, even in patients with poor or diminished fixation ability and/or with an irregular cornea surface.

**15.** Apparatus according to any one of claims 1 to 14, **characterised by** a microscope whose observing partial beam path or observing beam paths observing the object **(24.8; 24.9; 24.10)** is or are a component part of the measuring arm of the measuring device, and coupling of observing partial beam path and partial beam path of the measuring arm by at least one beam splitter **(129a, 129b; 137a, 137b)** takes place.

## Revendications

**1.** Dispositif comprenant une source de rayonnement laser (7, 89, 93) pour l'ablation de matière de la surface d'un objet (24.1 à 24.10) transparent ou diffus du point de vue de la technique du rayonnement, avec lequel une pluralité de procédures consécutives d'ablation partielle selon la technique du rayonnement peut être entreprise, avant et/ou après chaque procédure d'ablation partielle la distance relative de la zone d'ablation partielle par rapport à une valeur de référence conjointement avec l'épaisseur effective de l'objet est déterminable automatiquement par interférométrie de Michelson et uniquement dans la mesure où une valeur de distance spécifiée est déterminable à l'intérieur d'une tolérance, en fonction de la valeur d'épaisseur respectivement déterminée une valeur d'ablation partielle suivante est spécifiable automatiquement, de telle sorte qu'après la fin de l'ablation un profil d'épaisseur spécifié ou un profil d'épaisseur bi et/ou tridimensionnel de l'objet traité (24.1 à 24.12) peut être obtenu, le dispositif comprenant un dispositif de commande (3), une source de rayonnement laser (7) commandée par le dispositif de commande (11), un dispositif de mesure (1, 1.1 à 1.6) avec un interféromètre de Michelson et une unité d'évaluation électronique (41, 50.1, 50.2) raccordée selon les signaux au dispositif de commande (3), laquelle est configurée de telle sorte qu'elle détermine, à partir des valeurs de mesure déterminées avec le dispositif de mesure (1, 1.1 à 1.6), la distance relative de la zone d'ablation partielle par rapport à une valeur de référence conjointement avec l'épaisseur de l'objet et à partir de cette dernière le profil d'épaisseur spécifié ou le profil d'épaisseur bi et/ou tridimensionnel de l'objet (24.1 à 24.10) et compte tenu du profil d'épaisseur déterminé commande la source de rayonnement laser, celle-ci étant uniquement déclenchée dans la mesure où le dispositif de commande a déterminé une valeur de distance enregistrée située dans une plage de tolérances spécifiée et/ou en particulier une inclinaison de la zone d'ablation partielle à l'intérieur de valeurs de tolérance enregistrées, **caractérisé en ce que** le dispositif de mesure (1.1. à 1,6) pourvu de l'interféromètre de Michelson possède une source de rayonnement de mesure (32.1 à 32.4) avec une longueur d'onde centrale, la longueur d'onde centrale se situant dans une largeur de bande de $\pm$100 nm pour une longueur d'onde de 1310 nm, et dans le bras de référence (30 ;49b ; 56a, 56b, 32.3 ; 144) est utilisée une fibre monomodale (35, 74, 76) pour cette longueur d'onde, afin que le longueur du chemin optique dans l'air dans le bras de mesure (31 ; 49a ; 54) qui est nécessaire pour la réalisation de la mesure soit parfaitement compensable.

**2.** Dispositif selon la revendication 1, **caractérisé en ce que** le dispositif de mesure (1, 1.1 à 1.6) comprend un variateur de longueur de chemin et l'unité d'évaluation comprend au moins une mémoire, la longueur de variation du chemin étant au moins aussi importante que l'épaisseur à mesurer de la cornée encore non traitée, et au moins une valeur d'épaisseur (DMF) de la cornée non traitée est enregistrée dans la mémoire en tant que différence d'une première longueur de variation de chemin d'une première interférence apparaissant concernant la face arrière de la cornée et d'une seconde longueur de variation de chemin d'une seconde interférence apparaissant concernant la face avant de la cornée, et au moins une autre valeur d'épaisseur (DOF) de la cornée avec le lambeau cornéen (flap) découpé et déplié dans la cornée.

**3.** Dispositif selon la revendication 2, **caractérisé en ce qu'**une valeur d'épaisseur de la cornée avec le lambeau cornéen (flap) découpé, mais pas encore déplié, est en outre déterminable avec le dispositif de mesure et enregistrable dans la mémoire.

**4.** Dispositif selon la revendication 1, **caractérisé en ce que** le dispositif de mesure (1, 1.1 à 1.6) comprend un variateur de longueur de chemin et l'unité d'évaluation comprend au moins une mémoire, la longueur de variation de chemin étant au moins aussi importante que l'épaisseur à mesurer de la cornée et au moins une valeur d'épaisseur (DME) de la cornée est enregistrable dans la mémoire en tant que différence d'une première longueur de variation de chemin d'une première interférence apparaissant concernant la face arrière de la cornée et d'une seconde longueur de variation de chemin d'une seconde interférence apparaissant concernant la face avant de la cornée, et au moins une autre valeur d'épaisseur (DOE) de la cornée avec épithélium raclé.

**5.** Dispositif selon la revendication 2 à 4, **caractérisé en ce qu'**après une première ablation de matière avec une première impulsion laser sur la cornée sans lambeau cornéen et/ou sans épithélium, une troisième valeur d'épaisseur est déterminable et enregistrable dans la mémoire, pour obtenir une valeur indicative d'ablation (DAB) pour chaque impulsion laser suivante, et la troisième valeur d'épaisseur (MD) déterminable est comparable avec l'unité d'évaluation à une valeur d'épaisseur (KD) critique enregistrable dans la mémoire.

**6.** Dispositif selon une des revendications 1 à 5, **ca-**

**ractérisé par** une unité de déflexion de faisceau avec laquelle, étant commandable par le dispositif de commande, le faisceau laser de la source de rayonnement de mesure peut être défléchi sur la surface de l'objet.

**7.** Dispositif selon une des revendications 1 à 5, **caractérisé par** une unité de déflexion de faisceau avec laquelle, étant commandable par le dispositif de commande, le faisceau laser de la source de rayonnement de mesure peut être défléchi linéairement sur une cornée oculaire en tant que surface d'objet.

**8.** Dispositif selon une des revendications 1 à 5, **caractérisé par** une unité de déflexion de faisceau (19, 67) avec laquelle, étant commandable par le dispositif de commande, le faisceau laser de la source de rayonnement de mesure peut être défléchi, en étant commandé par le dispositif de commande, sur la surface d'objet, en plan pour la détermination d'un profil d'épaisseur tridimensionnel de l'objet.

**9.** Dispositif selon une des revendications 1 à 8, **caractérisé en ce que** l'interféromètre de Michelson du dispositif de mesure (1.4 ; 1.5) possède un bras de référence et un bras de mesure, le bras de mesure ayant une unité de séparation de faisceau multiple (85, 102) avec laquelle le faisceau de mesure (101) est divisable en plusieurs faisceaux de mesure, et l'interféromètre possède un détecteur configuré comme un réseau de détecteurs (105) pour les interférences entre le rayonnement de référence et le rayonnement de mesure.

**10.** Dispositif selon une des revendications 1 à 8, **caractérisé en ce que** l'interféromètre de Michelson du dispositif de mesure (1.4 ; 1.5) possède un bras de référence et un bras de mesure, le bras de mesure ayant un réseau de collimateurs (102) avec lequel le faisceau de mesure (101) est divisable en plusieurs faisceaux de mesure, et l'interféromètre possède un détecteur configuré comme un réseau de détecteurs (105) pour les interférences entre le rayonnement de référence et le rayonnement de mesure.

**11.** Dispositif selon la revendication 9 ou 10, **caractérisé par** un faisceau de fibres (86a, 86b; 103) avec lequel le faisceau de mesure (101) séparable en plusieurs faisceaux de mesure par l'unité de séparation de faisceau multiple (85, 102) ou par le réseau de collimateurs (102) est guidable sur l'objet pour la détermination du profil d'épaisseur ou pour la détermination de profil bi et/ou tridimensionnelle.

**12.** Dispositif selon une des revendications 1 à 11, **caractérisé en ce que** l'unité de déflexion de faisceau est conçue de telle sorte que le faisceau de mesure de l'interféromètre de Michelson est incident, jusqu'à une tolérance angulaire dans des positions déviant de l'axe optique (65), perpendiculairement également sur une surface convexe vers l'extérieur de l'objet.

**13.** Dispositif selon la revendication 12, **caractérisé en ce que** l'unité de déflexion de faisceau est conçue sous forme d'un cylindre creux (45.1 à 45.5) à couche réfléchissante à l'intérieur.

**14.** Dispositif selon les revendications 12 ou 13, **caractérisé en ce que** la surface convexe vers l'extérieur est la surface cornéenne (24.4), l'épaisseur cornéenne étant mesurable dans une zone la plus large possible autour du centre de la cornée même chez les patients souffrant d'une capacité de fixation déficiente et/ou réduite et/ou d'une surface cornéenne irrégulière.

**15.** Dispositif selon une des revendications 1 à 14, **caractérisé par** un microscope dont la trajectoire de faisceau de séparation d'observation observant l'objet (24.8 ;24.9 ; 24.10) ou les trajectoires de faisceau d'observation est ou respectivement sont un élément partiel du bras de mesure du dispositif de mesure, et un couplage entre la trajectoire de faisceau de séparation d'observation et la trajectoire de faisceau de séparation du bras de mesure est effectué par au moins un séparateur de faisceau (129a, 129b ; 137a, 137b).

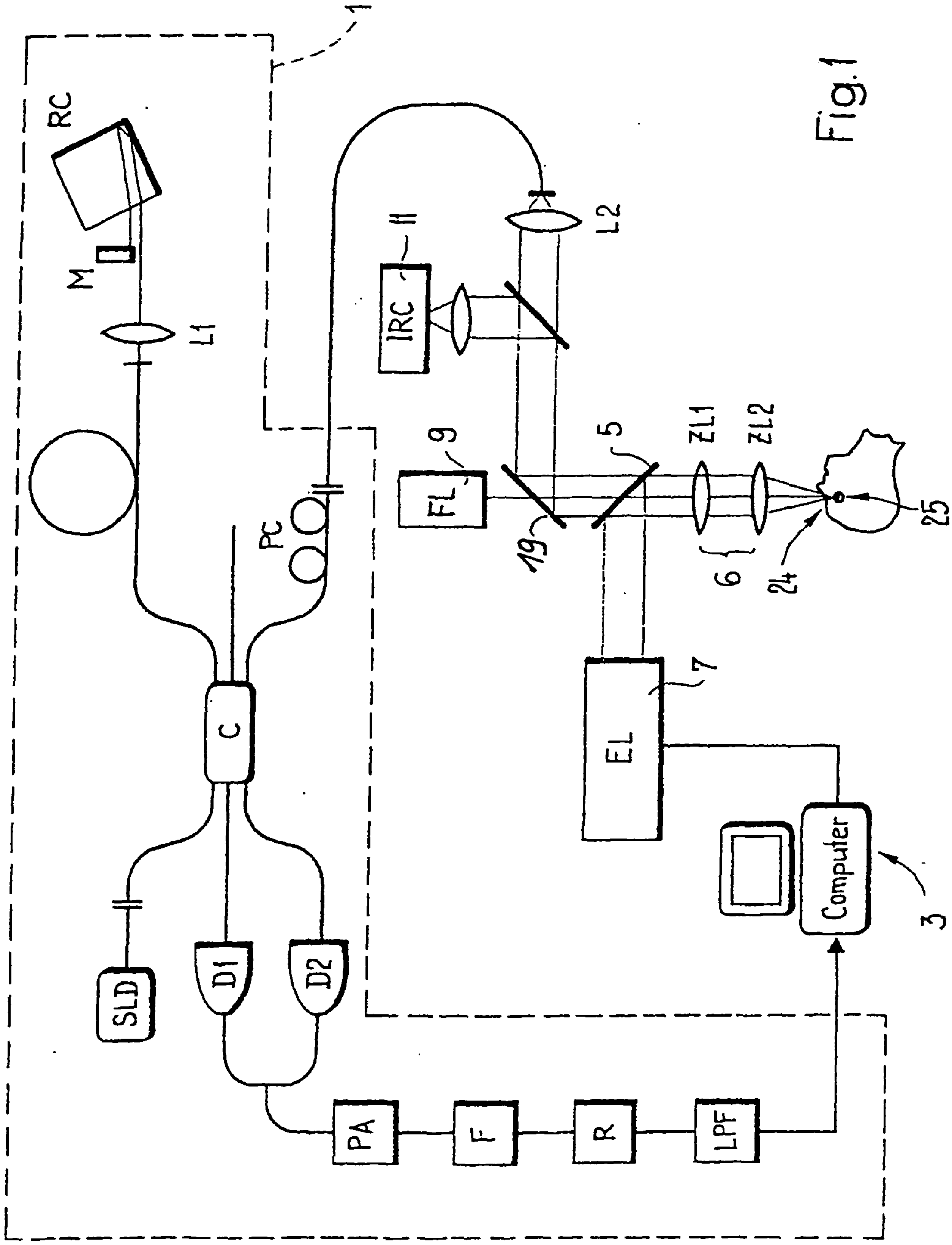
1
RC
M
L1
L2
IRC
11
PC
C
SLD
D1
D2
FL
9
19
5
ZL1
ZL2
6
24
25
7
EL
Computer
3
PA
F
R
LPF
Fig.1

Fig.2

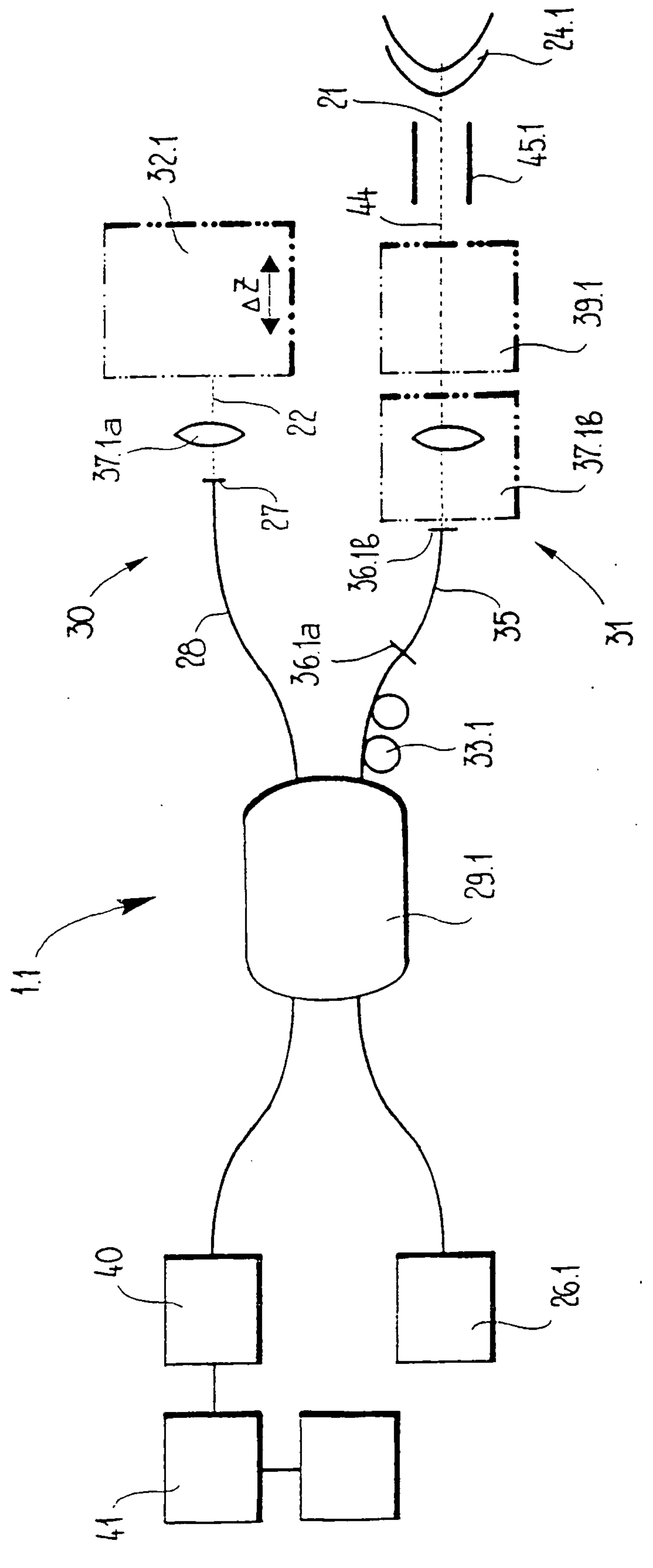
24.1
21
45.1
44
32.1
Δz
39.1
22
37.1a
37.1b
27
36.1b
30
28
36.1a
35
31
33.1
29.1
1.1
40
26.1
41

Fig.3

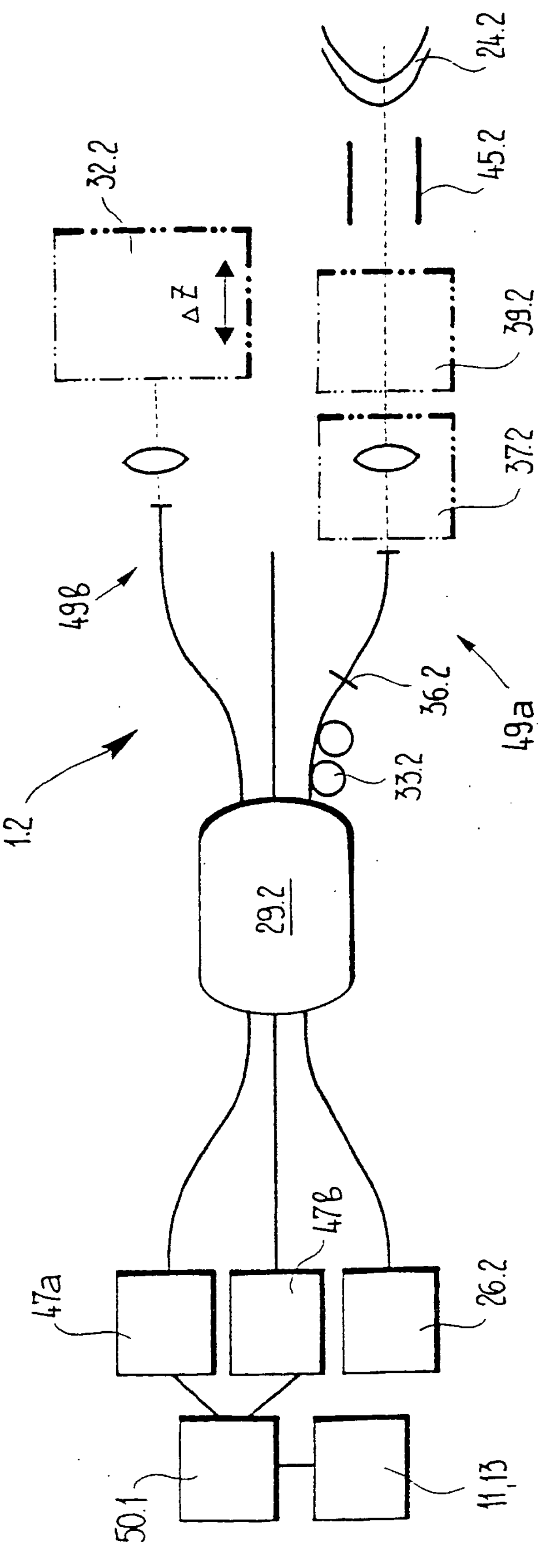
32.2
ΔZ
24.2
45.2
39.2
37.2
49b
49a
36.2
33.2
1.2
29.2
47a
47b
26.2
50.1
11.13

Fig.4

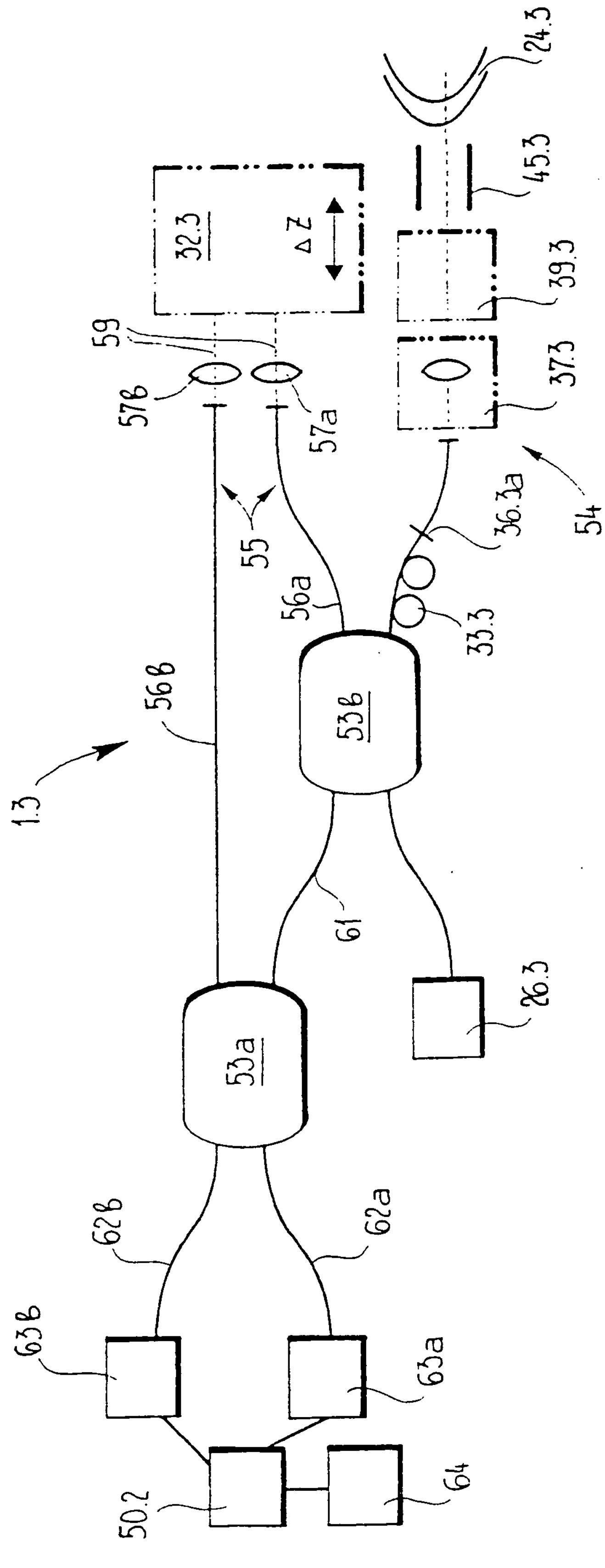
1.3
56b
53a
53b
32.3
ΔZ
59
57b
57a
55
56a
33.3
36.3a
54
37.3
39.3
45.3
24.3
61
26.3
62b
62a
63b
63a
50.2
64

Fig.5

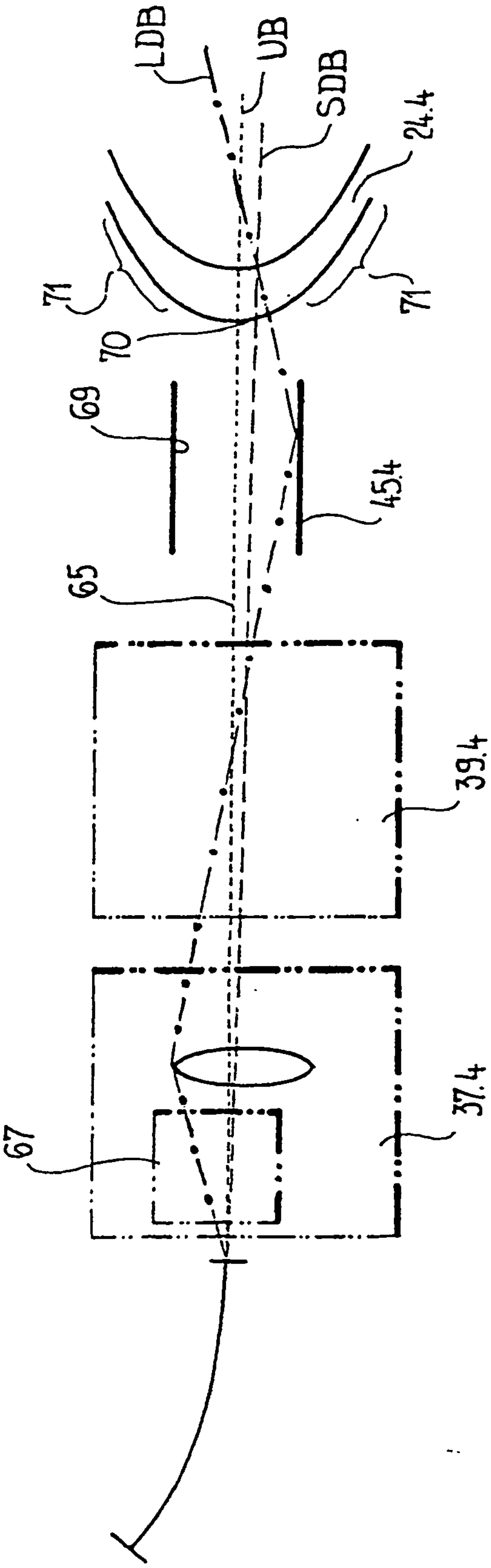

Fig.6

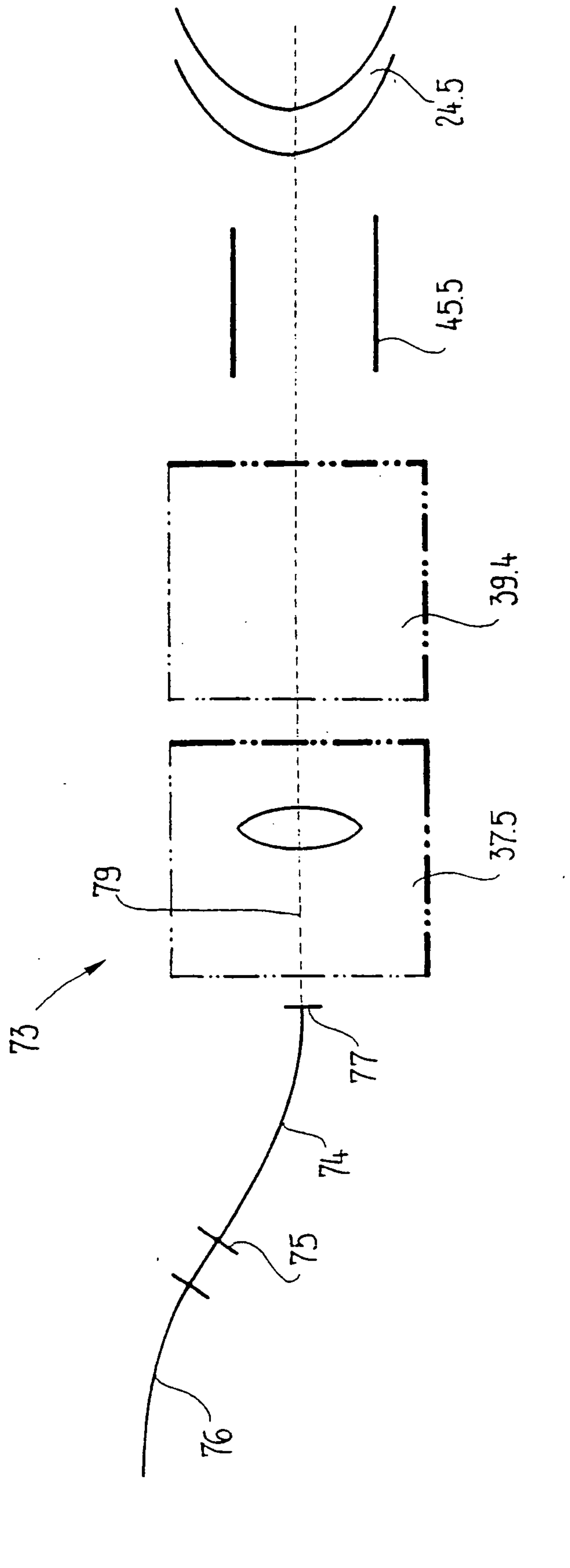

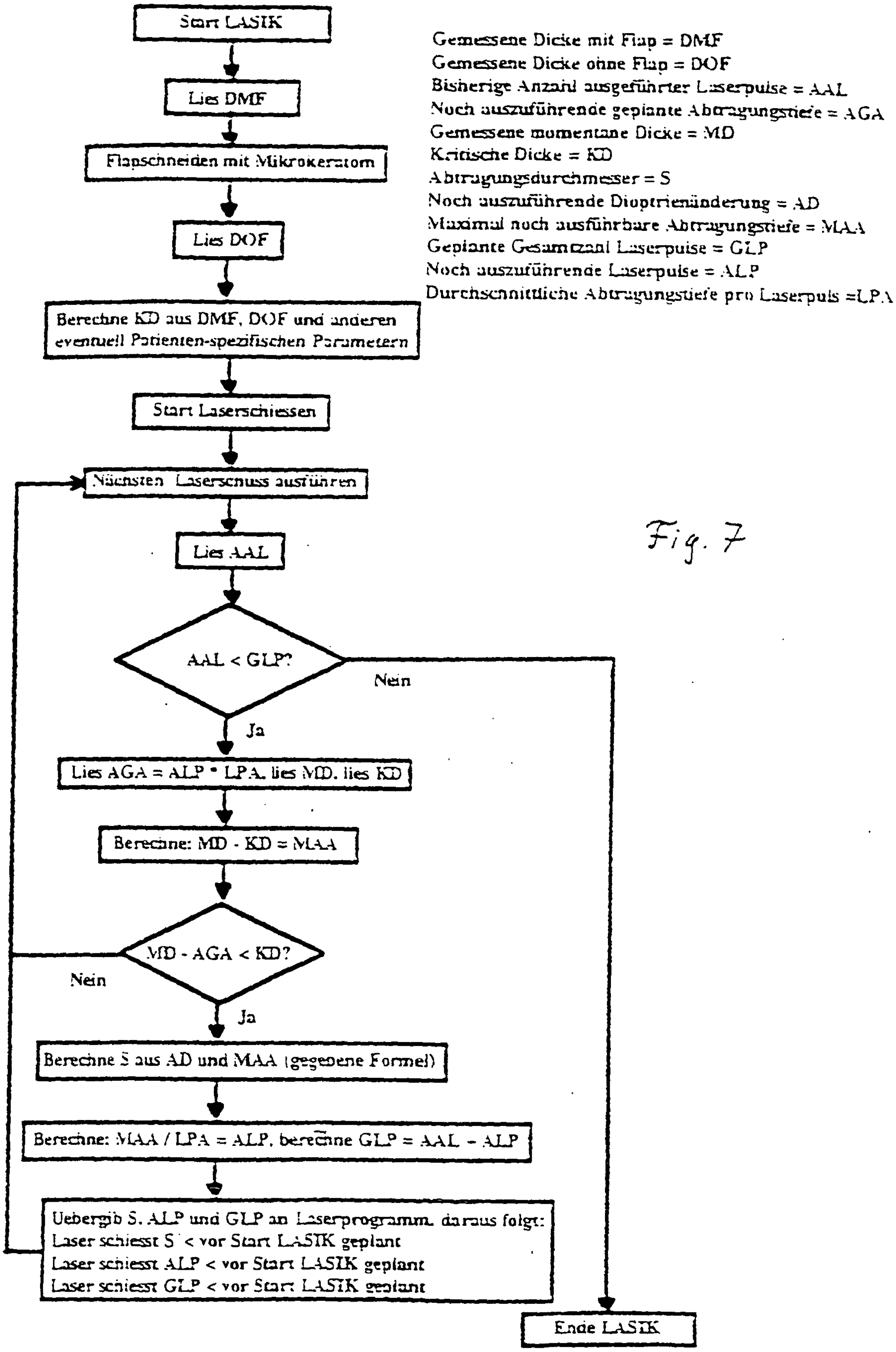
Start LASIK
Lies DMF
Flapschneiden mit Mikrokeratom
Lies DOF
Berechne KD aus DMF, DOF und anderen eventuell Patienten-spezifischen Parametern
Start Laserschiessen
Nächsten Laserschuss ausführen
Lies AAL
AAL < GLP?
Nein
Ja
Lies AGA = ALP * LPA, lies MD, lies KD
Berechne: MD - KD = MAA
MD - AGA < KD?
Nein
Ja
Berechne S aus AD und MAA (gegebene Formel)
Berechne: MAA / LPA = ALP, berechne GLP = AAL - ALP
Uebergib S, ALP und GLP an Laserprogramm, daraus folgt:
Laser schiesst S < vor Start LASIK geplant
Laser schiesst ALP < vor Start LASIK geplant
Laser schiesst GLP < vor Start LASIK geplant
Ende LASIK
Gemessene Dicke mit Flap = DMF
Gemessene Dicke ohne Flap = DOF
Bisherige Anzahl ausgeführter Laserpulse = AAL
Noch auszuführende geplante Abtragungstiefe = AGA
Gemessene momentane Dicke = MD
Kritische Dicke = KD
Abtragungsdurchmesser = S
Noch auszuführende Dioptrienänderung = AD
Maximal noch ausführbare Abtragungstiefe = MAA
Geplante Gesamtzahl Laserpulse = GLP
Noch auszuführende Laserpulse = ALP
Durchschnittliche Abtragungstiefe pro Laserpuls = LPA

Fig. 7

Gemessene Dicke mit Epithel = DME
Gemessene Dicke ohne Epithel = DOE
Bisherige Anzahl ausgeführter Laserpulse = AAL
Noch auszuführende geplante Abtragungstiefe = AGA
Gemessene momentane Dicke = MD
Kritische Dicke = KD
Abtragungsdurchmesser = S
Noch auszuführende Dioptrienänderung = AD
Maximal noch ausführbare Abtragungstiefe = MAA
Geplante Gesamtzahl Laserpulse = GLP
Noch auszuführende Laserpulse = ALP
Durchschnittliche Abtragungstiefe pro Laserpuls =LPA

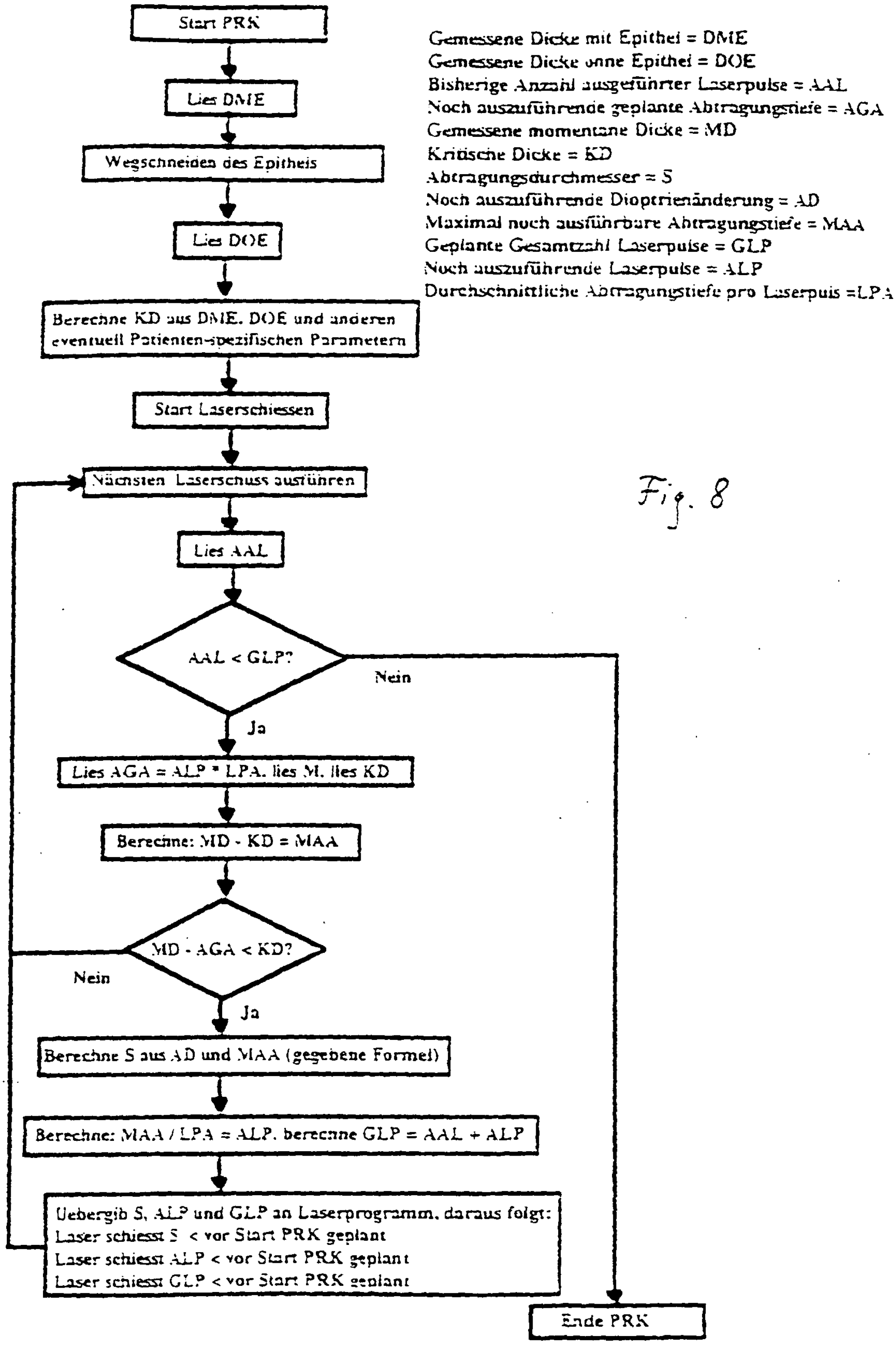


Fig. 8

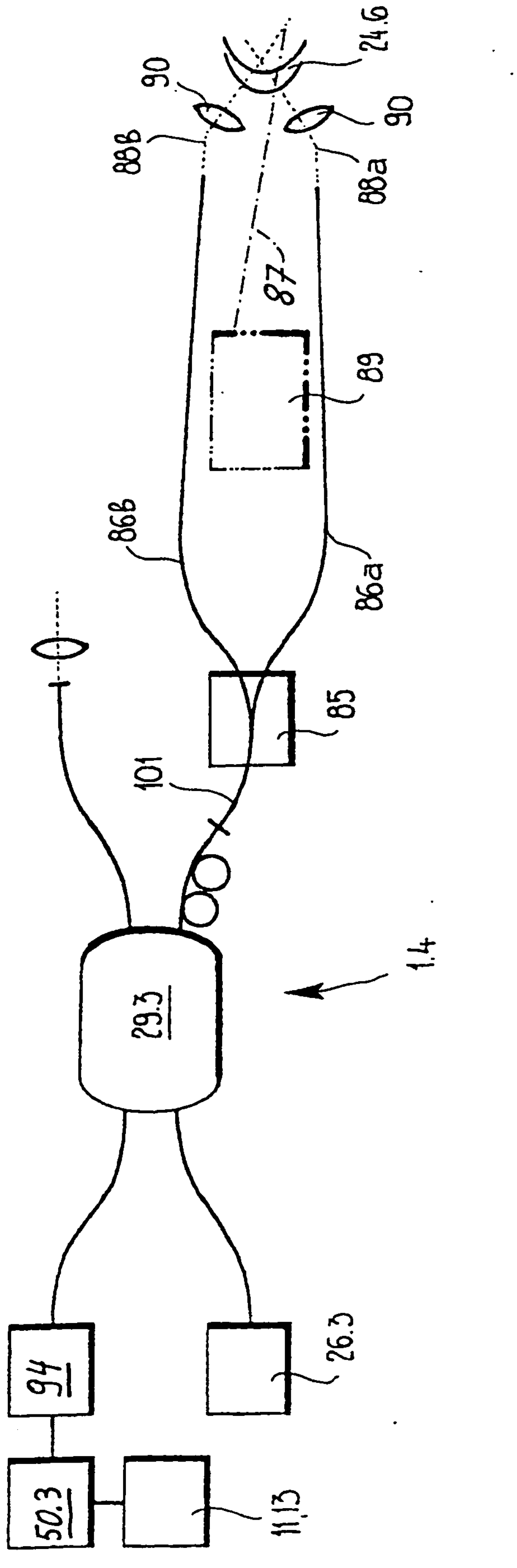


Fig.9

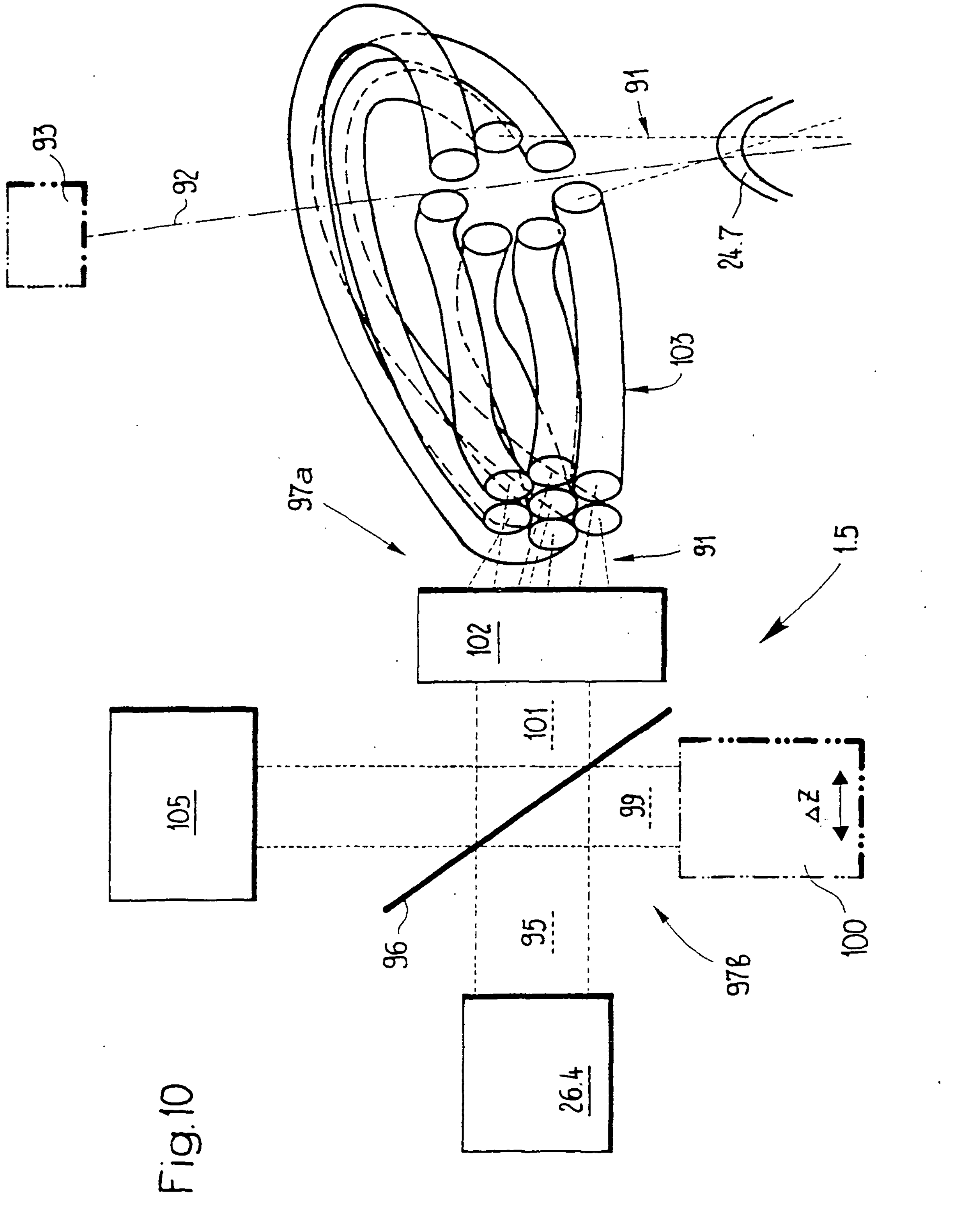


Fig.10

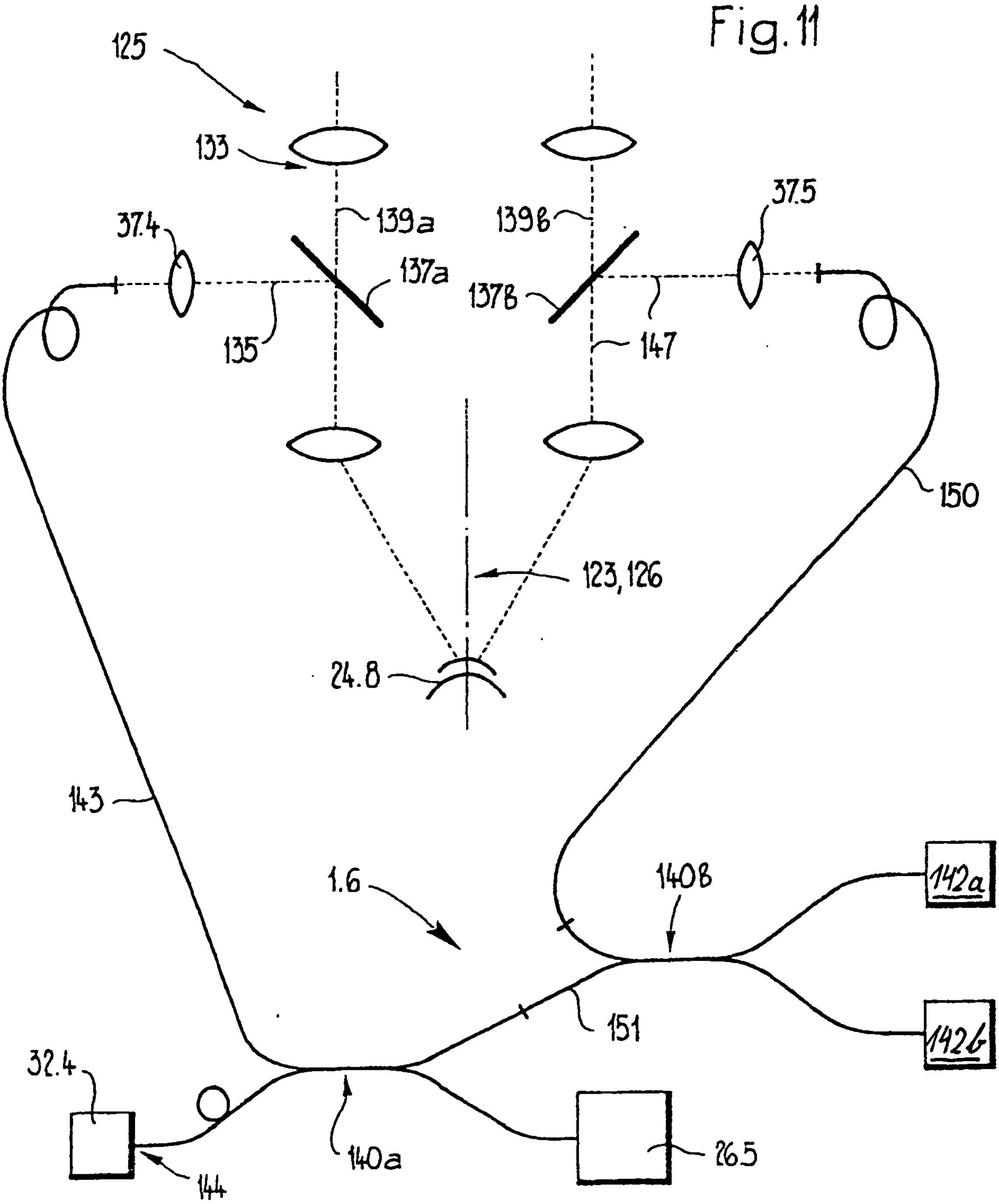
Fig.11
125
133
37.4
139a
139b
37.5
137a
137b
135
147
150
123,126
24.8
143
1.6
140b
142a
151
142b
32.4
140a
26.5
144

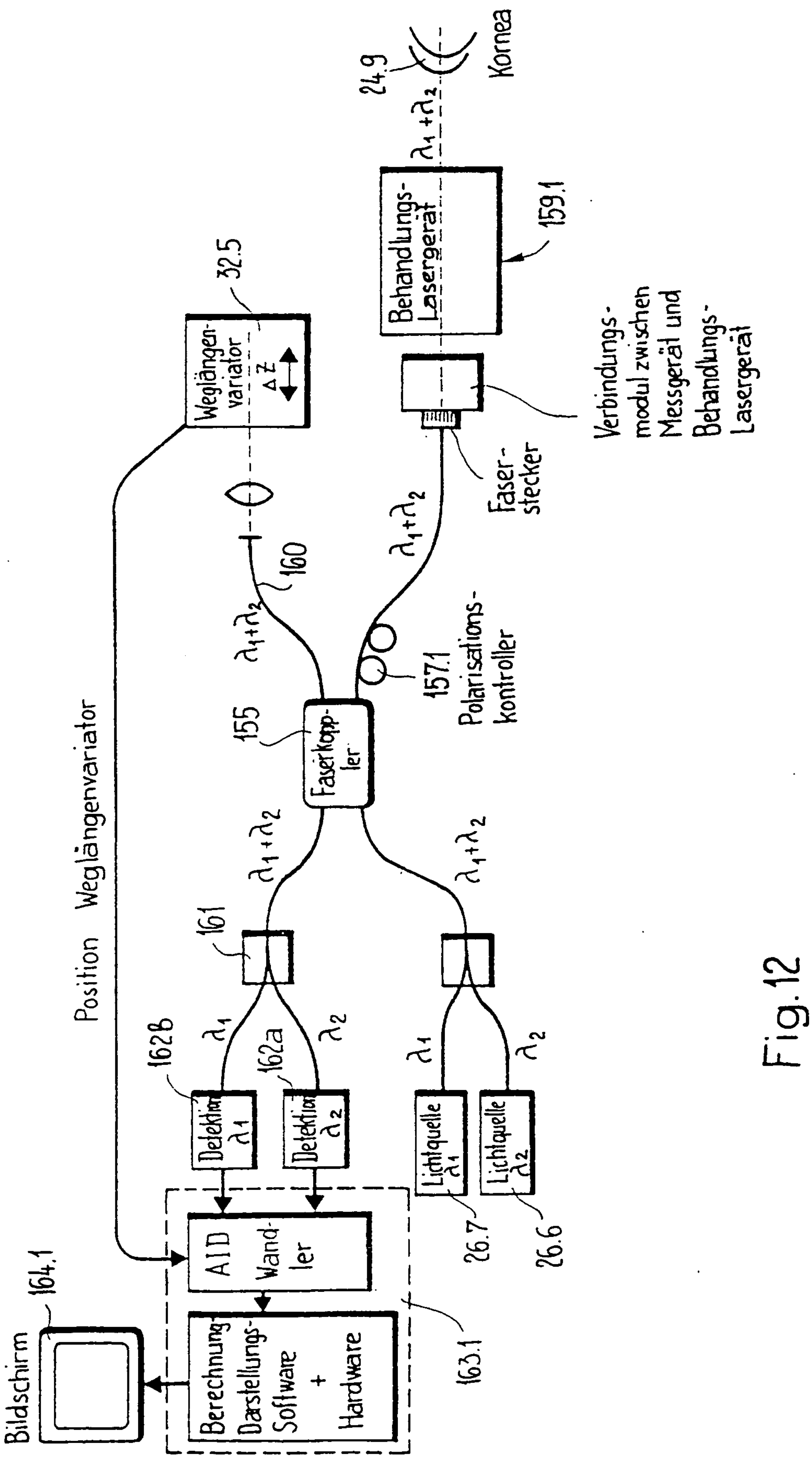
Kornea
24.9
$\lambda_1+\lambda_2$
Behandlungs-Lasergerät
159.1
Weglängen-variator
$\Delta z$
32.5
Verbindungs-modul zwischen Messgerät und Behandlungs-Lasergerät
Faser-stecker
$\lambda_1+\lambda_2$
160
$\lambda_1+\lambda_2$
157.1
Polarisations-kontroller
155
Faserkoppler
$\lambda_1+\lambda_2$
$\lambda_1+\lambda_2$
161
Position Weglängenvariator
162b
162a
$\lambda_1$
$\lambda_2$
$\lambda_1$
$\lambda_2$
Detektion $\lambda_1$
Detektion $\lambda_2$
Lichtquelle $\lambda_1$
Lichtquelle $\lambda_2$
26.7
26.6
A/D Wandler
Berechnungs- Darstellungs- Software + Hardware
163.1
Bildschirm
164.1

Fig.12

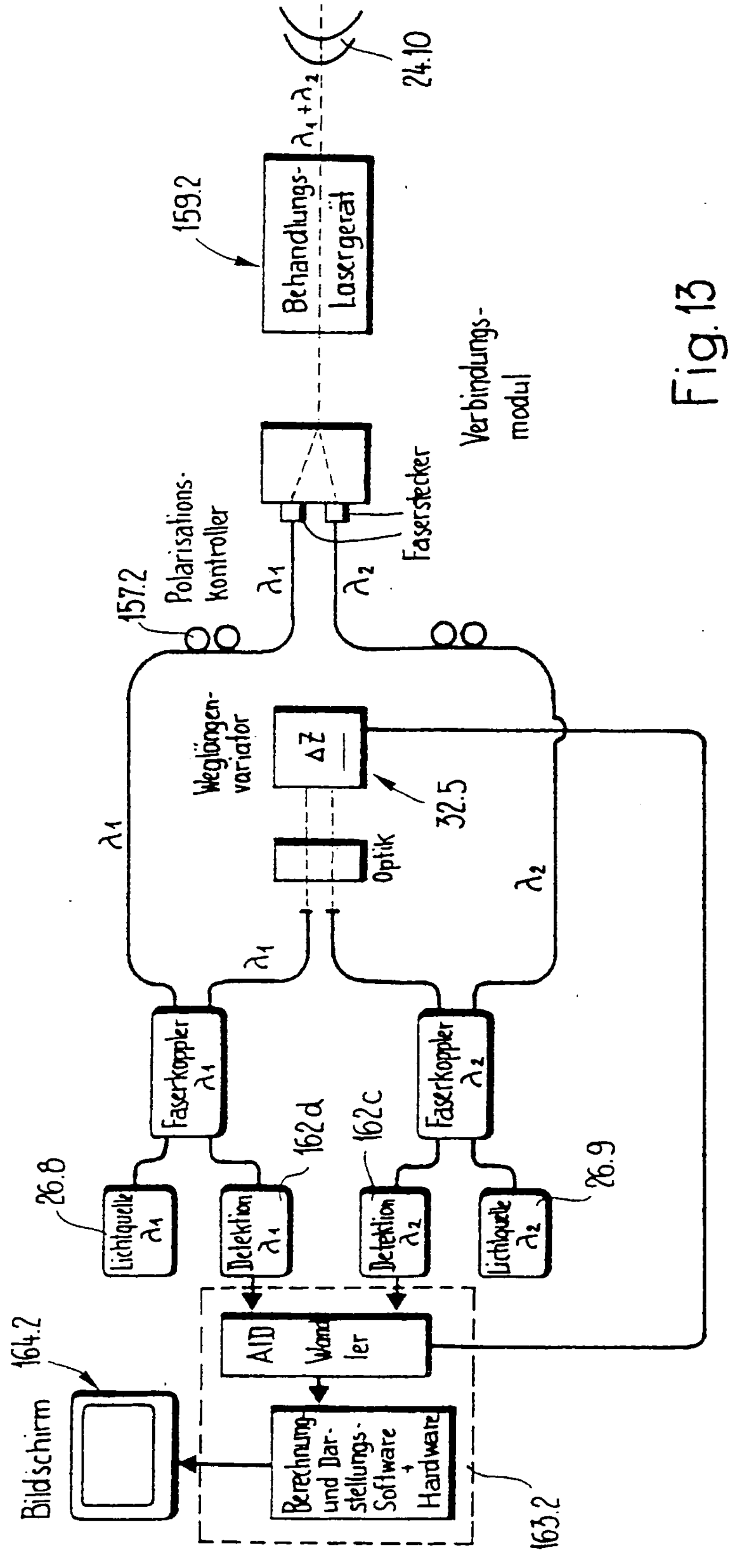
24.10
λ1 + λ2
159.2
Behandlungs-Lasergerät
Verbindungs-modul
Faserstecker
Polarisations-kontroller
157.2
λ1
λ2
Weglängen-variator
ΔZ
32.5
Optik
λ1
λ2
Faserkoppler λ1
Faserkoppler λ2
162d
162c
26.8
26.9
Lichtquelle λ1
Detektion λ1
Detektion λ2
Lichtquelle λ2
A/D Wandler
Berechnung und Darstellungs-Software + Hardware
163.2
Bildschirm
164.2

Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 5720894 A
- US 5693109 A
- DE 19704602 A
- WO 9922198 A

### In der Beschreibung aufgeführte Nicht-Patentliteratur

- **JAY H. KRACHMER ; MARK J. MANNIS ; EDWARD J. HOLLAND.** *Surgery of the Cornea and Conjunctiva,* 1997, vol. III